# FASCICULE DE BREVET EUROPEEN

(11) **EP 4 349 311 B1**
(45) Date de publication et mention de la délivrance du brevet: **27.08.2025**
(21) Numéro de dépôt: 23201545.3
(22) Date de dépôt: 04.10.2023
(51) Int. Cl.: A61F 13/02

(54) **PANSEMENT À FILM MINCE POURVU DE PERFORATIONS ET DE FENTES**
DÜNNFILMVERBAND MIT PERFORATIONEN UND SCHLITZEN
THIN FILM DRESSING WITH PERFORATIONS AND SLITS

(30) Priorité: 05.10.2022 FR 2210212
(43) Date de publication de la demande: 10.04.2024
(73) Titulaire: Gergonne SAS, 01100 Oyonnax (FR)
(72) Inventeur: GERGONNE, Charles, 01100 ARBENT (FR); GERGONNE, Bertrand, 01100 ARBENT (FR)
(74) Mandataire: Germain Maureau

(56) Documents cités:
- EP-B1- 2 773 304
- FR-A1- 3 102 056
- US-A- 4 541 426
- US-A- 5 633 007
- US-A1- 2019 000 677

## Description

La présente invention concerne un pansement à film mince pourvu de perforations et de fentes.

Dans le domaine des pansements souples et de faible épaisseur, dits « pansements à film mince », qui sont généralement utilisés comme couche de protection sur des plaies (notamment des plaies de grands brûlés), il est courant que ces pansements comprennent au moins une couche constituée d'un film mince et souple de polymère ou de copolymère (par exemple un film de polyuréthane) et qui présente les propriétés avantageuses suivantes :
- du fait de sa finesse et de sa souplesse, une extensibilité dans toutes les directions qui rend le pansement conformable et donc adaptable à la partie anatomique (à savoir parcelle de peau ou ongle) sur laquelle il est appliqué et notamment au niveau d'une articulation ;
- une haute perméabilité à la vapeur d'eau et une imperméabilité aux liquides (tels que l'eau, les solvants et les liquides corporels) qui fait également obstacle à la contamination bactérienne, le tout favorisant la cicatrisation dans un environnement humide. Il s'agit de « l'effet barrière » du pansement.

Ces pansements à film mince peuvent être aussi utilisés pour fixer des cathéters, des sondes de mesure ou des appareils électroniques portatifs.

Dans le cadre de la présente invention, on entend par « partie anatomique » aussi bien une parcelle de peau qu'un ongle. La description qui suit fera plus souvent référence à la peau sans que cela ne limite la portée de l'invention à l'application du pansement sur la peau. La portée de l'invention s'étend donc aussi à l'application du pansement sur un ongle.

Dans le cadre de la présente invention, on entend par « film mince et souple de polymère ou copolymère », un film à base de polymère ou copolymère que l'on peut fléchir, plier et courber facilement sans qu'il ne se casse ou ne se détériore. Ledit film présente une souplesse telle qu'il peut se conformer de manière appropriée pour s'adapter à la courbure de la partie anatomique sur laquelle est appliqué le pansement, et notamment au niveau d'une articulation. Son épaisseur peut être comprise entre 2 µm et 2000 µm, de préférence entre 5 µm et 500 µm, plus préférentiellement entre 10 µm et 200 µm, encore plus préférentiellement entre 10 µm et 70 µm, et de manière tout à fait préférée entre 10 µm et 30 µm.

Les pansements à film mince comprennent une couche de film mince et souple de polymère ou copolymère dont une des faces est recouverte d'une couche adhésive (par exemple une couche d'adhésif à base d'acrylique ou de gel de silicone). Une couche de protection détachable (par exemple une couche de polyester enduite d'une fine couche de silicone ou de fluorosilicone) recouvre généralement la face de la couche adhésive qui n'est pas en contact avec la couche de film mince et souple de polymère ou copolymère.

En outre, pour certains de ces pansements à film mince, de multiples perforations peuvent être présentes dans la couche de film mince et souple de polymère ou copolymère pour améliorer sa respirabilité, ou autrement dit améliorer sa perméabilité à la vapeur d'eau.

Cependant, ces pansements à film mince, même perforés, ne sont pas toujours optimaux pour le confort de son utilisateur. En effet, ces pansements à film mince ne sont pas toujours parfaitement conformables sur certaines parties anatomiques, telles que par exemple les coudes. De plus, cette conformabilité peut être amoindrie lorsque la partie anatomique sur laquelle a été déposée le pansement est mise en mouvement. Le pansement à film mince a alors tendance à se décoller de la peau. le document US2019000677 décrit un tel pansement.

De plus, cette conformabilité du pansement peut être réduite lors d'efforts physiques (par exemple musculaires) qui génèrent de la transpiration. En effet, si la sueur sortant des pores de la parcelle de peau recouverte par le pansement n'est pas évacuée, cela va contribuer à ce que le pansement se décolle.

Enfin, si le pansement n'est pas parfaitement conformable sur la peau, il peut se former sur ledit pansement, et notamment lors de mouvements de son utilisateur (entraînant éventuellement l'évacuation de sueur), des plis, aussi appelés « tunnels », qui s'étendent d'une extrémité du pansement à sa partie centrale et qui sont à l'origine de la rupture de la barrière bactérienne que le pansement est censé garantir.

Les inventeurs de la présente invention ont cherché à surmonter ces problèmes connus de manque de conformabilité des pansements à film mince et des inconvénients détaillés ci-dessus que cela peut engendrer, en mettant au point un nouveau pansement à film mince :
- parfaitement conformable, et ce quels que soient la partie anatomique concernée et les mouvements (éventuellement accompagnés de transpiration) de son utilisateur,
- présentant un effet barrière tel que défini ci-dessus (à savoir une haute perméabilité à la vapeur d'eau et une imperméabilité aux liquides) qui est excellent,
- ne comportant pas de risque de formation de plis (autrement dit « des tunnels ») tels que détaillés ci-dessus et à l'origine de la rupture de la barrière bactérienne.

La présente invention a ainsi pour objet un pansement à film mince qui comprend au moins :
- un 1^{er} film mince et souple de polymère ou copolymère présentant une 1^{ère} face et une 2^{ème} face ;
- une couche d'un 1^{er} adhésif dont une des deux faces est en contact avec la 1^{ère} face du 1^{er} film mince et souple de polymère ou copolymère ;
- une couche de protection détachable positionnée sur la face de la couche du 1^{er} adhésif qui est opposée à celle qui est en contact avec la 1^{ère} face du 1^{er} film mince et souple de polymère ou copolymère ;
ledit pansement se caractérise en ce que :
il comprend en outre :
   - une couche d'un 2^{ème} adhésif dont une des deux faces est en contact avec la 2^{ème} face du 1^{er} film mince et souple de polymère ou copolymère,
   - un 2^{ème} film mince et souple de polymère ou copolymère présentant une 1^{ère} face et une 2^{ème} face, la 1^{ère} face de ce 2^{ème} film mince et souple de polymère ou copolymère étant en contact avec la face de la couche du 2^{ème} adhésif qui est opposée à celle en contact avec la 2^{ème} face du 1^{er} film mince et souple de polymère ou copolymère ;
l'ensemble constitué par la couche de 1^{er} adhésif, le 1^{er} film mince et souple de polymère ou copolymère et la couche de 2^{ème} adhésif comporte des perforations et des fentes qui s'étendent dans toute l'épaisseur dudit ensemble.

Dans le cadre de la présente invention, les perforations et les fentes sont deux types d'ouvertures clairement différents.

A la différence des perforations, les fentes sont des ouvertures étroites et allongées qui ont été pratiquées dans l'ensemble constitué par la couche de 1^{er} adhésif, le 1^{er} film mince et souple de polymère ou copolymère et la couche de 2^{ème} adhésif, et ce avec un minimum de retrait de matières de cet ensemble, de préférence avec aucun retrait de matières.

Les perforations sont des ouvertures qui ont été pratiquées dans ledit ensemble avec un retrait de matières de cet ensemble.

A la différence des perforations, les fentes sont des ouvertures longilignes.

A la différence des fentes, les perforations peuvent présenter toute forme polygonale.

Les perforations présentent toujours un taux d'ouverture quel que soit l'état dudit ensemble. Ce taux d'ouverture peut plus ou moins augmenter en fonction de la force de l'étirement qui est appliqué sur ledit ensemble. Par contraste avec les perforations, lorsque ledit ensemble est étiré avec une force plus ou moins importante, les fentes vont passer d'un état « quasi fermé » dans lequel leur taux d'ouverture est très faible, voire nul, à un état « ouvert » dans lequel l'ouverture desdites fentes s'est agrandie.

Ainsi, le pansement selon l'invention présente les avantages suivants :
- l'association tout à fait originale de fentes et de perforations dans l'ensemble constitué par la couche de 1^{er} adhésif, le 1^{er} film mince et souple de polymère ou copolymère et la couche de 2^{ème} adhésif permet non seulement d'améliorer sa respirabilité (autrement dit la perméabilité à la vapeur d'eau) grâce auxdites perforations mais également sa conformabilité grâce auxdites fentes,
- en outre, l'association dudit ensemble perforé et fendu avec un 2^{ème} film mince et souple de polymère ou copolymère garantit un effet barrière tel que défini ci-dessus qui est excellent. En effet, le 2^{ème} film mince et souple de polymère ou copolymère, de par ses propriétés intrinsèques, garantit cet effet barrière,
- grâce à son excellente conformabilité, lors de mouvements de son utilisateur (éventuellement accompagnés de transpiration), l'absence de formation de plis (ou autrement dit de « tunnels ») tels que définis ci-dessus sur ledit pansement et qui sont à l'origine d'une rupture de la barrière bactérienne. En d'autres termes, la barrière bactérienne est assurée avec le pansement selon l'invention, et ce quels que soient la partie anatomique sur laquelle est appliqué le pansement et les mouvements (éventuellement avec de la transpiration) de son utilisateur.

Autrement dit, l'originalité du pansement selon l'invention réside dans l'association de deux films minces et souples de polymère ou copolymère dont le 1^{er} est perforé et fendu dans toute son épaisseur pour permettre une excellente respirabilité et conformabilité et le 2^{ème} garantissant un effet barrière tel que défini ci-dessus.

Il est à noter qu'une bonne respirabilité du pansement est très avantageuse car elle permet d'éviter une accumulation de sueur sous le pansement ce qui génère un inconfort du patient et le décollement du pansement.

Le pansement à film mince selon l'invention est particulièrement approprié en tant que couche de protection sur des plaies (notamment des plaies de grands brûlés). Il peut aussi parfaitement être utilisé pour fixer des cathéters, des sondes de mesure ou des appareils électroniques portatifs.

La surface du pansement peut être comprise entre 5 cm² et 800 cm², de préférence entre 16 cm² et 450 cm².

Le pansement peut présenter toutes formes. Il peut s'agir notamment d'une forme carrée, rectangulaire, ronde ou ovale. Par exemple, le pansement peut présenter une forme carrée dont le côté est compris entre 25 mm et 300 mm, de préférence entre 40 mm et 200 mm. Dans un mode de réalisation de l'invention, le côté du carré mesure 100 mm. Par exemple, le pansement peut présenter une forme rectangulaire dont la largeur est comprise entre 10 mm et 200 mm, de préférence entre 30mm et 150 mm et la longueur est comprise entre 50 mm et 400 mm, de préférence entre 60 mm et 300 mm. Dans un mode de réalisation de l'invention, la largeur mesure 100 mm et la longueur mesure 300 mm.

Les 1^{er} et 2^{ème} films minces et souples de polymère ou copolymère présentent les caractéristiques techniques qui ont été décrites en introduction de la présentation de l'invention.

L'épaisseur de ces 1^{er} et 2^{ème} films minces et souples de polymère ou copolymère peut être comprise entre 2 µm et 2000 µm, de préférence entre 5 µm et 500 µm, plus préférentiellement entre 10 µm et 200 µm, encore plus préférentiellement entre 10 µm et 70 µm.

Plus précisément, l'épaisseur du 1^{er} film mince et souple de polymère ou copolymère peut être comprise entre 15 µm et 150 µm, de préférence entre 30 µm et 100 µm. L'épaisseur du 2^{ème} film mince et souple de polymère ou copolymère peut être comprise entre 5 µm et 50 µm, de préférence entre 10 µm et 30 µm.

De manière avantageuse, l'épaisseur du 1^{er} film mince et souple de polymère ou copolymère est plus importante que celle du 2^{ème} film mince et souple de polymère ou copolymère. Par exemple, le ratio de l'épaisseur du 1^{er} film mince et souple de polymère ou copolymère sur l'épaisseur du 2^{ème} film mince et souple de polymère ou copolymère peut être compris entre 1,5 et 10, de préférence entre 3 et 6.

Les 1^{er} et 2^{ème} films minces et souples de polymère ou copolymère sont choisis de manière à se conformer à la surface de la partie anatomique sur laquelle est fixé le pansement selon l'invention, et notamment au niveau des articulations, et ce même lorsque cette surface est en mouvement. Lesdits films présentent ainsi une certaine résilience pour continuer à se conformer à la surface de la partie anatomique lors de mouvements de flexion et d'extension.

Les 1^{er} et 2^{ème} films minces et souples de polymère ou copolymère peuvent, par exemple, être choisis parmi les films de polyuréthane, polyester, polyamide, polyéther, polychlorure de vinyle, polychlorure de vinylidène, d'alcools polyvinyliques, polyacétate de vinyle, polystyrène, polyoléfines (telles que polyéthylène et polypropylène), fluorure polyvinylique, les élastomères de silicone, ou encore parmi les films de copolymère de polyéther-polyester, polyester-polyuréthane, polyéther-polyuréthane, polyéther-polyamide, ainsi que les films de copolymères triblocs ou diblocs de styrène et d'oléfine (par exemple styrène/butadiène) et les films de polyéthers bloc amides.

Le 1^{er} et le 2^{ème} films minces et souples de polymère ou copolymère peuvent être réalisés en des matériaux (par exemple choisis parmi ceux décrits ci-dessus) identiques ou différents.

Des exemples de film mince et souple de polymère ou copolymère qui sont appropriés dans le cadre de la présente invention sont décrits dans les brevets US 5,088,483 et US 5,160,315.

De manière préférée, les 1^{er} et 2^{ème} films minces et souples de polymère ou copolymère sont transparents ou translucides, ou bien légèrement colorés tout en conservant une certaine transparence. Cela facilite la pose du pansement sur la partie anatomique (à savoir parcelle de peau ou ongle).

Les 1^{er} et 2^{ème} films minces et souples de polymère ou copolymère sont perméables à la vapeur d'eau. La perméabilité à la vapeur d'eau du film mince et souple d'une épaisseur de 20 µm est avantageusement supérieure à 200 g/m²/24 heures, plus préférentiellement supérieure à 1000 g/m²/24 heures et encore plus préférentiellement supérieure à 5000 g/m²/24 heures. Par exemple, elle peut être comprise entre 100 et 50000 g/m²/24 heures, de préférence entre 1000 et 40000 g/m²/24 heures, plus préférentiellement entre de 5000 à 30000 g/m²/24 heures.

Les 1^{er} et 2^{ème} films minces et souples de polymère ou copolymère peuvent être obtenus à partir d'une solution de polyuréthane commercialisée par la société ICAP-SIRA S.p.a. sous les dénominations commerciales « ICAFLEX BR447 MATT 10 » et « ICAFLEX BR447 MATT 3/25 ». Après séchage, le film ainsi obtenu est un film de polyuréthane.

De manière préférée, les 1^{er} et 2^{ème} films minces et souples de polymère ou copolymère sont des films de polyuréthane. En effet, ils présentent l'avantage d'être respirant et d'évacuer la vapeur d'eau sur la peau.

La couche de 1^{er} adhésif est réalisée en un matériau adhésif pouvant être utilisé sur le corps. Il est avantageusement hypoallergénique.

De préférence, la couche de 1^{er} adhésif présente une haute perméabilité à la vapeur d'eau.

En outre, la couche de 1^{er} adhésif présente une adhérence élevée sur la partie anatomique sur laquelle est fixée le pansement selon l'invention, et notamment le cas échéant pour sécuriser la pose d'un cathéter.

La couche de 1^{er} adhésif peut être un adhésif sensible à la pression (aussi connu sous la dénomination anglophone de « Pressure Sensitive Adhesive »). Plus précisément, la couche de 1^{er} adhésif peut être un adhésif sensible à la pression à base d'acrylique, de polyuréthane, de silicone, de caoutchouc naturel, de copolymère d'éthylène et d'acétate de vinyle ou de copolymère blocs du type poly(styrène-isoprène-styrène).

Des exemples d'adhésifs sensibles à la pression sont décrits dans les brevets EP 0 035 399 B1, EP 0 051 935 B1, US 3,389,827, US 4,112,213, US 4,310,509, US 4,323,557 et US 4,737,410.

La couche de 1^{er} adhésif peut également être une couche d'un adhésif sensible à la pression à base d'acrylique commercialisé par la société ASHLAND sous les dénominations commerciales AROSET 1920-Z-52 ou AROSET 1910-TH-52.

La couche de 1^{er} adhésif peut également être une couche d'un adhésif sensible à la pression à base de caoutchouc commercialisé par la société H.B. FULLER sous la dénomination commerciale SwiftCol 9063.

Dans d'autres modes de réalisation de l'invention, la couche de 1^{er} adhésif peut être un gel choisi parmi le gel de silicone, le gel de polyuréthane et les hydrogels.

De préférence, la couche de 1^{er} adhésif comprend du gel de silicone qui procure d'excellentes propriétés d'adhérence sur la peau du pansement selon l'invention lors de sa pose. En outre, le gel de silicone présente l'avantage de rendre atraumatique le retrait du pansement. Enfin, si des parties de la couche de 1^{er} adhésif se retrouvent collées ensemble, du fait qu'il s'agit de gel de silicone, elles peuvent être redécollées facilement sans risque d'abîmer le 1^{er} film mince et souple de polymère ou copolymère qui est très fin.

Le gel de silicone est un composé de silicone sous forme réticulée. Il procure une adhérence élevée à la peau et une faible force de libération par décollement. De préférence, le pouvoir adhésif du gel de silicone est compris entre 0,5 N/cm et 4 N/cm. Le gel de silicone présente une cohésion telle qu'il ne laisse pas de résidus sur la peau lors du retrait du pansement tout en restant accroché au 1^{er} film mince et souple de polymère ou copolymère dont il est solidaire.

Le gel de silicone peut être fabriqué à partir de précurseurs de silicone qui réticulent après leur mise en contact suivant une réaction d'hydrosilylation ou de condensation. Le gel de silicone peut être obtenu à partir d'un mélange d'une résine de poly-di-organosiloxane et de catalyseur, par exemple dans un ratio en poids de ces composés précités de 1 : 1, 3 : 1 ou encore de 10 : 1, ledit mélange de poly-di-organosiloxane et de catalyseur étant soumis à une réticulation. Un exemple de gel de silicone est décrit dans le brevet US 4 991 574.

Par exemple, le gel de silicone est choisi parmi les produits de :
- la société WACKER qui sont dénommés SILPURAN, série 2120,
- la société ELKEM de la série SILBIONE 4717,
- la société NUSIL qui sont dénommés CEREPLAST MED 634.

Le procédé de réticulation du gel de silicone est parfaitement à la portée de l'homme du métier.

L'épaisseur de la couche de 1^{er} adhésif peut être comprise entre 5 µm et 500 µm, de préférence entre 10 µm et 250 µm.

La masse surfacique de la couche de 1^{er} adhésif peut être comprise entre 5 g/m² et 500 g/m², de préférence, entre 10 g/m² et 300 g/m². Les adhésifs sensibles à la pression à base d'acrylique ont des masses surfaciques plus faibles (à savoir inférieures à 50 g/m²) que celles des gels de silicone qui sont supérieures à 100 g/m².

La couche de 1^{er} adhésif peut en outre comprendre au moins un composé choisi parmi les agents antimicrobiens. Cela présente l'avantage de favoriser la cicatrisation, et lorsqu'un cathéter est disposé sous le pansement de contrôler une infection sur cette zone potentiellement à risque infectieux.

La couche de 2^{ème} adhésif peut être réalisée en un matériau adhésif à base d'acrylique, de silicone, de polyuréthane, de caoutchouc naturel, d'hydrogel, de copolymère d'éthylène et d'acétate de vinyle ou de copolymère blocs du type poly(styrène-isoprène-styrène). De préférence, la couche de 2^{ème} adhésif est une couche d'un adhésif acrylique.

L'épaisseur de la couche de 2^{ème} adhésif peut être comprise entre 5 µm et 100 µm, de préférence entre 10 µm et 50 µm.

Comme expliqué ci-dessus, le pansement selon l'invention a pour originalité de comprendre un ensemble constitué par la couche de 1^{er} adhésif, le 1^{er} film mince et souple et la couche de 2^{ème} adhésif qui comporte des perforations et des fentes s'étendant dans toute son épaisseur.

Les caractéristiques techniques de ces perforations et fentes sont maintenant décrites plus en détail.

Le taux d'ouverture des perforations peut être compris entre 10% et 60%, de préférence entre 20% et 40%. Par « taux d'ouverture des perforations », on entend le pourcentage de la surface totale des perforations exprimé par rapport à la surface que présenterait ledit ensemble en l'absence de perforations. Si le taux d'ouverture est trop important, des problèmes de décollement du pansement selon l'invention peuvent être constatés.

Les perforations peuvent présenter toutes formes géométriques diverses et variées, par exemple circulaire, carrée, rectangulaire, triangulaire, ovale. De préférence, les perforations ont une forme circulaire. En effet, les perforations circulaires peuvent être aisément mises en œuvre au cours de la fabrication du pansement selon l'invention. Dans ce mode de réalisation de l'invention, le diamètre des perforations peut être compris entre 0,5 mm et 10 mm, de préférence entre 2 mm et 5 mm.

Les perforations peuvent avoir des formes identiques ou différentes entre elles.

Plus généralement, quelle que soit la forme géométrique des perforations, la surface de chaque perforation peut être comprise entre 0,2 mm² et 80 mm², de préférence entre 1,5 mm² et 20 mm². Ces intervalles de surface sont particulièrement appropriés pour procurer une bonne conformabilité au pansement et éviter tout problème de décollement intempestif.

Les perforations peuvent avoir des surfaces identiques ou différentes entre elles.

Dans des modes de réalisation de l'invention, les perforations peuvent être disposées de manière à être toutes alignées les unes avec les autres ou bien être toutes disposées en quinconce.

Dans d'autres modes de réalisation de l'invention, les perforations ne sont pas toutes alignées les unes avec les autres ou bien elles ne sont pas toutes en quinconce.

Les perforations peuvent être disposées de manière aléatoire (à savoir par exemple sans suivre aucun alignement) sur l'ensemble constitué par la couche de 1^{er} adhésif, le 1^{er} film mince et souple de polymère ou copolymère et la couche de 2^{ème} adhésif.

La disposition des perforations peut être homogène ou bien hétérogène sur ledit ensemble.

En d'autres termes, dans des modes de réalisation de l'invention, la surface dudit ensemble peut comprendre des zones plus ou moins denses en perforations.

Dans des modes de réalisation de l'invention, la répartition des perforations peut ne pas être uniforme sur la surface dudit ensemble.

Dans des modes de réalisation de l'invention, ledit ensemble peut comporter une ou plusieurs zones dans lesquelles les perforations peuvent être alignées les unes avec les autres ou bien être en quinconce ou encore être disposées de manière tout à fait aléatoire.

La disposition des perforations sur ledit ensemble peut être diverse et variée.

Les fentes peuvent avoir une longueur comprise entre 0,5 mm et 20 mm, de préférence entre 1 mm et 10 mmm. Ces intervalles de longueur de fentes sont particulièrement appropriés pour procurer une excellente conformabilité au pansement.

La largeur des fentes peut être inférieure à 2 mm, de préférence inférieure à 1 mm. De manière avantageuse, la largeur des fentes est la plus faible possible. Ainsi, de manière préférée, aucun matériau de l'ensemble constitué par la couche de 1^{er} adhésif, le 1^{er} film mince et souple de polymère ou copolymère et la couche de 2^{ème} adhésif n'a été retiré lors de la formation des fentes dans toute l'épaisseur dudit ensemble au cours de la fabrication du pansement selon l'invention.

Les fentes peuvent avoir des caractéristiques (par exemple la longueur et la largeur) identiques ou différentes entre elles.

Dans des modes de réalisation de l'invention, les fentes relient au moins 2 perforations adjacentes les unes des autres, plus préférentiellement au moins 4 perforations adjacentes les unes des autres. En effet, lorsque les fentes relient au moins 2 perforations adjacentes, de préférence entre 2 et 4 perforations adjacentes, cela améliore la conformabilité du pansement.

De manière avantageuse, la longueur totale des fentes par unité de surface dudit ensemble constitué par la couche de 1^{er} adhésif, le 1^{er} film mince et souple de polymère ou copolymère et la couche de 2^{ème} adhésif peut être comprise entre 1 mm/cm² et 30 mm/cm², de préférence entre 2 mm/cm² et 15 mm/cm². Ces intervalles sont particulièrement appropriés pour une bonne conformabilité du pansement selon l'invention.

La disposition des fentes peut être homogène ou bien hétérogène sur l'ensemble constitué par la couche de 1^{er} adhésif, le 1^{er} film mince et souple de polymère ou copolymère et la couche de 2^{ème} adhésif.

En d'autres termes, dans des modes de réalisation de l'invention, la surface dudit ensemble peut comprendre des zones plus ou moins denses en fentes.

Dans des modes de réalisation de l'invention, la répartition des fentes peut ne pas être uniforme sur la surface dudit ensemble.

La disposition des fentes sur ledit ensemble peut être diverse et variée.

Bien entendu dans le cadre de la présente invention, des caractéristiques et des dispositions des perforations et des fentes très variées peuvent être mises en œuvre. En d'autres termes, le pansement selon l'invention peut présenter des configurations très variées en ce qui concerne les perforations et les fentes qui lui procurent une excellente conformabilité et sans problème de décollement intempestif.

Dans un mode de réalisation de l'invention, le pansement comprend en outre une compresse qui est fixée :
- sur la face de la couche de 1^{er} adhésif qui est opposée à celle qui est en contact avec la 1^{ère} face du 1^{er} film mince et souple de polymère ou copolymère
   ou
- sur la face de la couche de 2^{ème} adhésif qui est opposée à celle qui est en contact avec la 2^{ème} face du 1^{er} film mince et souple de polymère ou copolymère.

Dans le mode de réalisation de l'invention dans lequel la compresse est fixée sur la couche de 1^{er} adhésif, ladite compresse peut recouvrir partiellement cette couche de 1^{er} adhésif de telle sorte que le pansement selon l'invention puisse être fixé sur le corps (à savoir une parcelle de peau ou ongle) grâce à ladite couche de 1^{er} adhésif.

Dans le mode de réalisation de l'invention dans lequel la compresse est fixée sur la couche de 2^{ème} adhésif, les exsudats de la plaie peuvent passer au travers les perforations dudit ensemble constitué par la couche de 1^{er} adhésif, le 1^{er} film mince et souple de polymère ou copolymère et la couche de 2^{ème} adhésif de manière à atteindre la compresse.

De manière préférée, la compresse est fixée au centre de ladite face de la couche de 1^{er} adhésif ou de ladite face de la couche de 2^{ème} adhésif.

La surface de la compresse peut être comprise entre 1 cm² et 480 cm², de préférence entre 4 cm² et 250 cm².

La compresse peut présenter toutes formes diverses et variées. Il peut s'agir d'une forme carrée, rectangulaire, ronde ou ovale. Par exemple, la compresse peut présenter une forme carrée dont le côté est compris entre 10 mm et 150 mm, de préférence entre 20 mm et 80 mm. Dans un mode de réalisation de l'invention, le côté du carré mesure 50 mm. Par exemple, la compresse peut présenter une forme rectangulaire dont la largeur est comprise entre 5 mm et 200 mm, de préférence entre 20 mm et 100 mm et la longueur est comprise entre 20 mm et 300 mm, de préférence entre 30 mm et 250 mm. Dans un mode de réalisation de l'invention, la largeur mesure 50 mm et la longueur mesure 250 mm.

L'épaisseur de la compresse peut être comprise entre 0,5 mm et 10 mm, de préférence entre 1,5 mm et 5 mm.

La compresse est réalisée en au moins un matériau absorbant.

Le matériau absorbant peut être choisi parmi :
- les mousses de polyuréthane, et plus préférentiellement les mousses de polyuréthane aliphatique,
- les non tissés tels que les non tissés en viscose ou à base d'un mélange de polyester et de viscose (par exemple, un mélange comprenant en poids de 70% à 80% de viscose et de 20 à 30% de polyester), ou encore les non tissés contenant de la cellulose,
- les matériaux en coton,
- les matériaux en cellulose.

Le choix du matériau absorbant est parfaitement à la portée de l'homme du métier.

La mousse de polyuréthane aliphatique présente l'avantage d'être une mousse absorbante souple à cellules ouvertes qui permet un excellent niveau de rétention d'environ 65 %. Elle présente également des capacités d'absorption de plus de 15 fois son poids. Son épaisseur peut varier de 1,5 à 5 mm. De plus, elle présente l'avantage de résister à l'irradiation des rayons bêta et gamma. Cela est particulièrement approprié lorsque le pansement selon l'invention est stérilisable ; ce qui implique que tous ses constituants doivent être stérilisables.

La compresse peut en outre comprendre au moins un composé choisi parmi les agents antimicrobiens.

Dans un mode de réalisation préféré de l'invention, la face de la compresse qui est opposée à celle qui est en contact avec la couche de 1^{er} adhésif (autrement dit la face de la compresse destinée à être en contact avec la partie anatomique), est recouverte en partie d'une couche d'un 3^{ème} adhésif. Par exemple, entre 20% et 80%, de préférence 50%, de la surface de cette face de la compresse est recouverte d'une couche de 3^{ème} adhésif. Cette couche de 3^{ème} adhésif est ainsi ajourée et peut par exemple présenter la forme d'un quadrillage.

Cette couche de 3^{ème} adhésif permet d'améliorer l'adhérence de la compresse sur la partie anatomique (peau ou ongle). En outre, le fait qu'elle soit ajourée permet le passage des exsudats de la plaie et garantit ainsi une bonne cicatrisation.

La couche de 3^{ème} adhésif peut être une couche de gel de silicone, un adhésif à base d'acrylique, de silicone, de polyuréthane, de caoutchouc naturel, d'hydrogel, de copolymère d'éthylène et d'acétate de vinyle ou de copolymère blocs du type poly(styrène-isoprène-styrène). De manière préférée, il s'agit d'une couche de gel de silicone.

Dans un mode de réalisation préféré de l'invention, 50% de la face de la compresse qui n'est pas en contact avec la couche de 1^{er} adhésif est recouverte par une couche de gel de silicone présentant la forme d'un quadrillage.

De manière préférée, le pansement selon l'invention est stérilisable. Cela signifie que tous ses constituants sont stérilisables. Cette propriété de stérilisation est indispensable si le pansement selon l'invention est destiné à être appliqué sur des plaies aigües, chroniques et exsudatives. Ces plaies sont souvent présentes sur des personnes affaiblies (par exemple des grands brûlés ou des personnes en fin de vie). La stérilisation du pansement est alors indispensable pour éviter tout risque de contamination.

Le matériau de la couche de protection détachable peut être choisi parmi les matériaux suivants :
- un film de polyester enduit d'une couche de fluorosilicone ou de silicone ;
- un film de polyoléfine tel que le polyéthylène, le polypropylène ou le polyéthylène téréphtalate ;
- une feuille métallique ;
- un papier enduit d'une couche de silicone, de fluorosilicone ou encore recouvert d'une couche de polyoléfine (par exemple du polypropylène ou du polyéthylène ou autre polyoléfine équivalente).

Par exemple, il peut s'agir d'un film polyéthylène qui est lisse ou bien encore embossé et/ou gaufré.

Le matériau de la couche de protection détachable est avantageusement un film de polyéthylène, car ce polymère par nature adhère peu aux couches d'adhésifs, notamment lorsqu'il s'agit d'un gel de silicone. De manière préférée, le film est un film de polyéthylène embossé et/ou gaufré afin de réduire les surfaces de contact avec la couche de 1^{er} adhésif et ainsi permettre son retrait aisé de la couche de 1^{er} adhésif lors de l'utilisation du pansement. Les films de polyéthylène gaufrés pointe de diamant de la société RKW, 55 µm et 65 µm, sont préférés.

L'épaisseur de la couche de protection détachable peut être comprise entre 30 µm et 150 µm. Dans un mode de réalisation de l'invention, cette épaisseur est de 60 µm.

La masse surfacique de la couche de protection détachable peut être comprise entre 25 g/m² et 120 g/m². De préférence, elle est de 50 g/m².

Dans un mode de réalisation de l'invention, la couche de protection détachable comporte deux parties qui se chevauchent dans la région médiane du pansement, de manière à permettre à l'utilisateur de les retirer sans toucher avec les doigts la couche de 1^{er} adhésif, donc sans lui faire perdre son pouvoir adhésif.

Dans un mode de réalisation de l'invention, la couche de protection détachable peut comprendre au moins une languette de préhension pour pouvoir la détacher de la couche de 1^{er} adhésif sans toucher ladite couche de 1^{er} adhésif et la contaminer.

La languette de préhension peut faire partie intégrante de la couche de protection détachable ou bien avoir été attachée à celle-ci, par exemple avec un adhésif ou un autre moyen de fixation.

Dans des modes de réalisation préférés du pansement selon l'invention, les 1^{er} et 2^{ème} films minces et souples sont en polyuréthane, la couche de 1^{er} adhésif est une couche de gel de silicone et la couche de 2^{ème} adhésif est une couche d'acrylique. Le pansement présente alors les avantages suivants :
- il est parfaitement conformable et respirant grâce aux fentes et aux perforations comme cela a été expliqué ci-dessus ;
- il est garant d'une excellente barrière aux bactéries comme cela a été expliqué ci-dessus ;
- grâce au gel de silicone, lorsque la couche de protection détachable a été retirée, si des parties de la couche de 1^{er} adhésif se retrouvent collées ensemble, elles peuvent être redécollées facilement sans risque d'abîmer les 1^{er} et 2^{ème} films minces et souples de polyuréthane qui sont très fins. De plus, il est repositionnable et son retrait est atraumatique.

Dans ce mode de réalisation préféré, ledit pansement peut en outre comprendre une compresse, par exemple une compresse telle que décrite ci-dessus, et de préférence réalisée en mousse de polyuréthane aliphatique. La compresse est fixée sur la face de la couche de 1^{er} adhésif qui est opposée à celle qui est en contact avec la 1^{ère} face du 1^{er} film mince et souple de polyuréthane ou sur la face de la couche de 2^{ème} adhésif qui est opposée à celle qui est en contact avec la 2^{ème} face du 1^{er} film mince et souple de polymère ou copolymère.

L'invention a aussi pour objet un procédé de fabrication du pansement tel que décrit ci-dessus. Le procédé de fabrication comprend au moins les étapes suivantes :
a) la 1^{ère} face du 1^{er} film mince et souple de polymère ou copolymère est recouverte de la couche de 1^{er} adhésif et la 2^{ème} face du 1^{er} film mince et souple de polymère ou copolymère est recouverte de la couche de 2^{ème} adhésif ;
b) une 1^{ère} couche de support est appliquée sur la face de la couche du 1^{er} adhésif qui est opposée à celle qui est en contact avec la 1^{ère} face du 1^{er} film mince et souple de polymère ou copolymère ;
c) une 2^{ème} couche de support est appliquée sur la face de la couche du 2^{ème} adhésif qui est opposée à celle qui est en contact avec la 2^{ème} face du 1^{er} film mince et souple de polymère ou copolymère ;
d) des perforations sont réalisées dans toute l'épaisseur d'un ensemble constitué par la 1^{ère} couche de support, la couche de 1^{er} adhésif, le 1^{er} film mince et souple de polymère ou copolymère, la couche de 2^{ème} adhésif et la 2^{ème} couche de support ;
e) des fentes sont réalisées dans toute l'épaisseur d'un ensemble constitué par la couche de 1^{er} adhésif, le 1^{er} film mince et souple de polymère ou copolymère, la couche de 2^{ème} adhésif et la 2^{ème} couche de support ;
f) la 2^{ème} couche de support est retirée ;
g) le 2^{ème} film mince et souple de polymère ou copolymère est appliqué sur la couche de 2^{ème} adhésif ;
h) la 1^{ère} couche de support est retirée ;
i) la couche de protection détachable est appliquée sur la couche de 1^{er} adhésif de manière à obtenir ledit pansement.

Le pansement ainsi obtenu peut être découpé aux dimensions souhaitées.

Les caractéristiques techniques du 1^{er} film mince et souple de polymère ou copolymère et des couches de 1^{er} et 2^{ème} adhésifs de l'étape a) ont été décrites ci-dessus.

L'épaisseur de la 1^{ère} couche de support de l'étape b) peut être comprise entre 30 µm et 150 µm. Dans un mode de réalisation de l'invention, cette épaisseur est de 60 µm.

Le matériau de la 1^{ère} couche de support peut être choisi parmi les matériaux suivants : polyéthylène, polyuréthane, polyester, polyamide, polyéther, polychlorure de vinyle, polychlorure de vinylidène, d'alcools polyvinyliques, polyacétate de vinyle, polystyrène, polyoléfines, fluorure polyvinylique, copolymère de polyéther-polyester, polyester-polyuréthane, polyéther-polyuréthane, polyéther-polyamide, les films de copolymères triblocs ou diblocs de styrène et d'oléfine, polyéthers bloc amides. De préférence, il s'agit d'une couche de polyéthylène. Avantageusement ce support peut être revêtu d'un revêtement anti-adhérent à base de silicone ou de fluorosilicone.

L'épaisseur de la 2^{ème} couche de support de l'étape c) peut être comprise entre 30 µm et 150 µm. Dans un mode de réalisation de l'invention, cette épaisseur est de 60 µm.

Le matériau de la 2^{ème} couche de support peut être choisi parmi les matériaux suivants : polyéthylène, polyuréthane, polyester, polyamide, polyéther, polychlorure de vinyle, polychlorure de vinylidène, d'alcools polyvinyliques, polyacétate de vinyle, polystyrène, polyoléfines, fluorure polyvinylique, copolymère de polyéther-polyester, polyester-polyuréthane, polyéther-polyuréthane, polyéther-polyamide, les films de copolymères triblocs ou diblocs de styrène et d'oléfine, polyéthers bloc amides.. De préférence, il s'agit d'une couche de polyéthylène téréphtalate. Avantageusement ce support peut être revêtu d'un revêtement anti-adhérent à base de silicone ou de fluorosilicone.

Les 1^{ère} et 2^{ème} couches de support qui sont appliquées respectivement aux étapes b) et c) permettent de protéger les couches respectivement de 1^{er} et 2^{ème} adhésifs pour les étapes suivantes du procédé de fabrication du pansement selon l'invention.

A l'étape e), la 1^{ère} couche de support qui recouvre la couche de 1^{er} adhésif n'est pas fendue et a donc uniquement été perforée lors de l'étape d). L'absence de fentes dans la 1^{ère} couche de support confère une certaine rigidité à l'ensemble constitué par la 1^{ère} couche de support, la couche de 1^{er} adhésif, le 1^{er} film mince et souple de polymère ou copolymère et la couche de 2^{ème} adhésif, en particulier lorsque la 2^{ème} couche de support sera retirée à l'étape f) pour pouvoir ensuite appliquer le 2^{ème} film mince et souple de polymère ou copolymère sur la couche de 2^{ème} adhésif à l'étape g).

A l'étape i), la couche de protection détachable présente les caractéristiques techniques qui ont été décrites ci-dessus.

Lorsque le pansement selon l'invention comprend en outre une compresse telle que décrite ci-dessus, son procédé de fabrication comprend une étape supplémentaire consistant, à l'issue de l'étape h) et avant l'étape i), à appliquer la compresse :
- sur la face de la couche de 1^{er} adhésif qui est opposée à celle qui est en contact avec la 1^{ère} face du 1^{er} film mince et souple de polymère ou copolymère
   ou
- sur la face de la couche de 2^{ème} adhésif qui est opposée à celle qui est en contact avec la 2^{ème} face du 1^{er} film mince et souple de polymère ou copolymère.

La compresse est une compresse telle que décrite ci-dessus.

Dans le mode de réalisation de l'invention dans lequel la compresse est appliquée sur la couche de 1^{er} adhésif, à l'étape i), la couche de protection détachable recouvre ainsi la compresse et la portion de la couche de 1^{er} adhésif qui n'est pas recouverte par ladite compresse.

L'invention sera mieux comprise à l'aide de la description détaillée qui est exposée ci-dessous en référence au dessin annexé représentant, à titre d'exemples non limitatifs, trois modes de réalisation de pansements à film mince selon l'invention, ainsi que des échantillons tests de film mince et souple de polyuréthane qui ont été utilisés pour des expérimentations.
[Fig. 1] La figure 1 est une vue en perspective et éclatée d'un pansement à film mince selon un 1^{er} mode de réalisation de l'invention.
[Fig. 2] La figure 2 est une vue du dessus de l'ensemble constitué par la couche du 2^{ème} adhésif, le 1^{er} film mince et souple de polymère ou copolymère et la couche de 1^{er} adhésif du pansement représenté sur la figure 1.
[Fig. 3] La figure 3 est une vue en coupe selon l'axe III-III de la figure 2 de l'ensemble constitué par la couche du 2^{ème} adhésif, le 1^{er} film mince et souple de polymère ou copolymère et la couche de 1^{er} adhésif du pansement représenté sur la figure 1.
[Fig. 4a] La figure 4a est une vue en perspective et éclatée d'un pansement à film mince selon un 2^{ème} mode de réalisation de l'invention.
[Fig. 4b] La figure 4b est une vue en perspective et éclatée d'un pansement à film mince selon un 3^{ème} mode de réalisation de l'invention.
[Fig. 5a] La figure 5a est une vue du dessus d'un 1^{er} échantillon test de 1^{er} film mince et souple de polyuréthane.

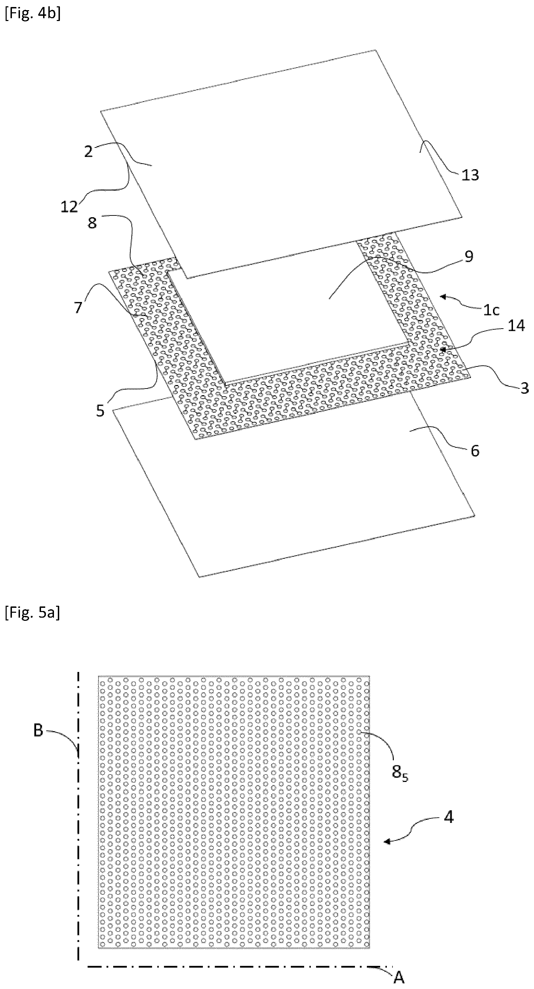

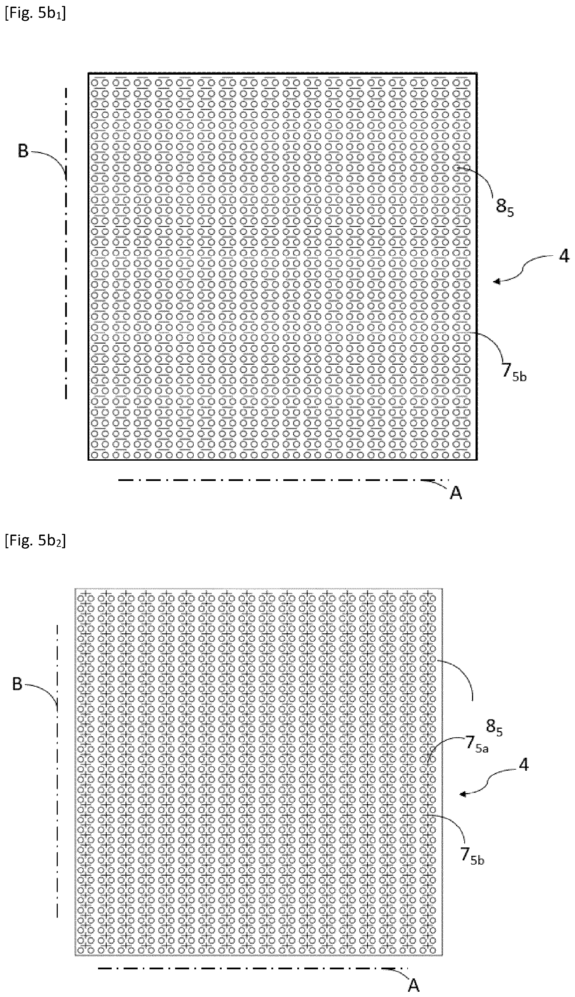

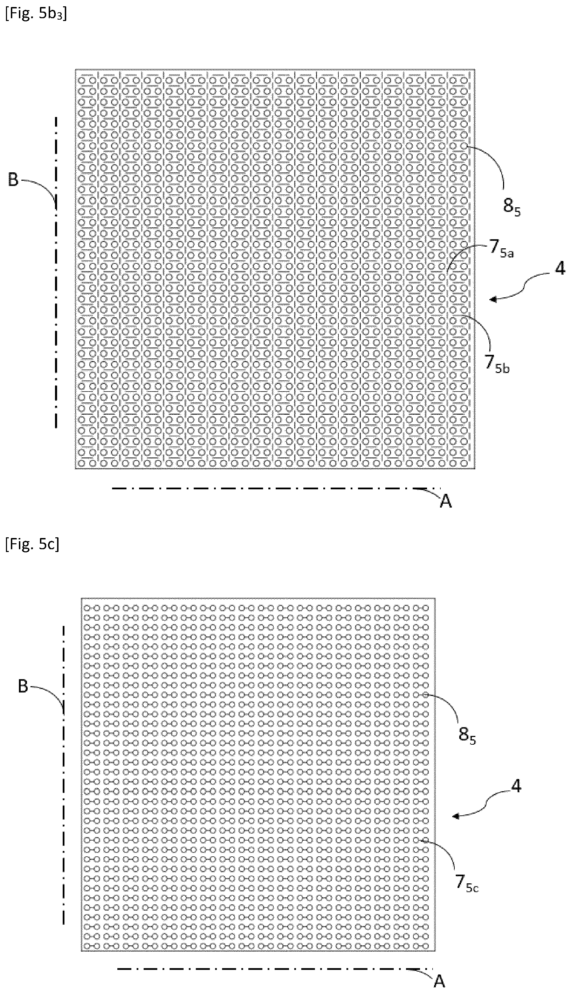

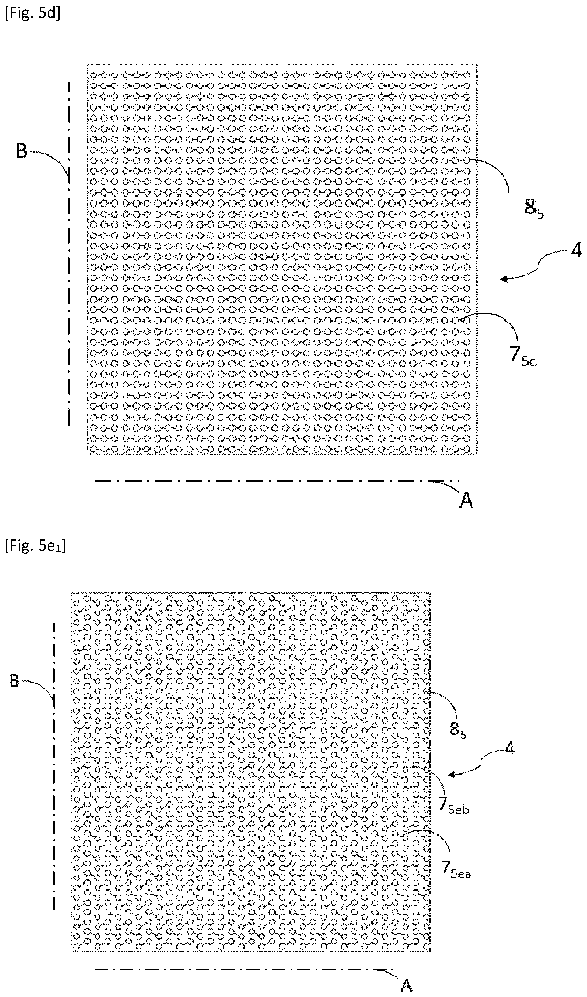

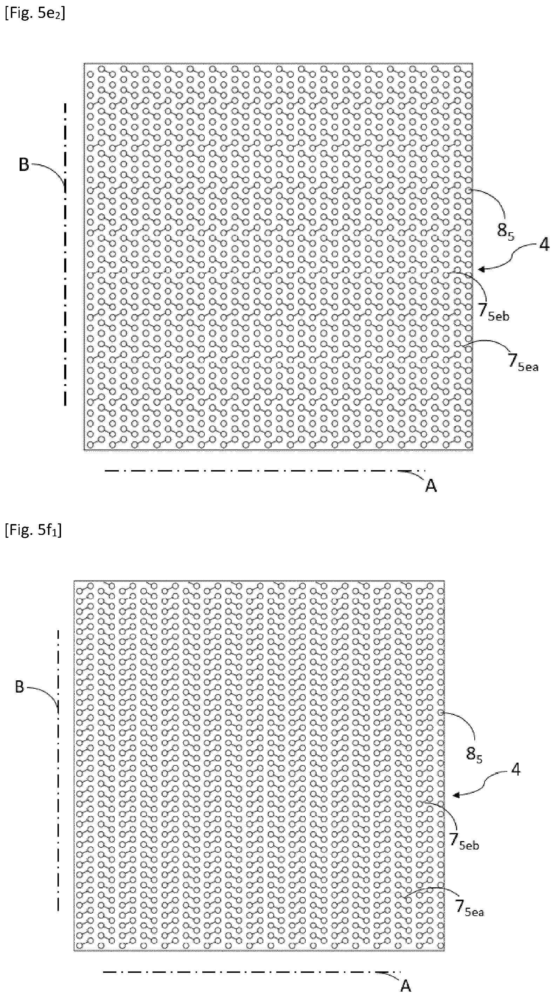

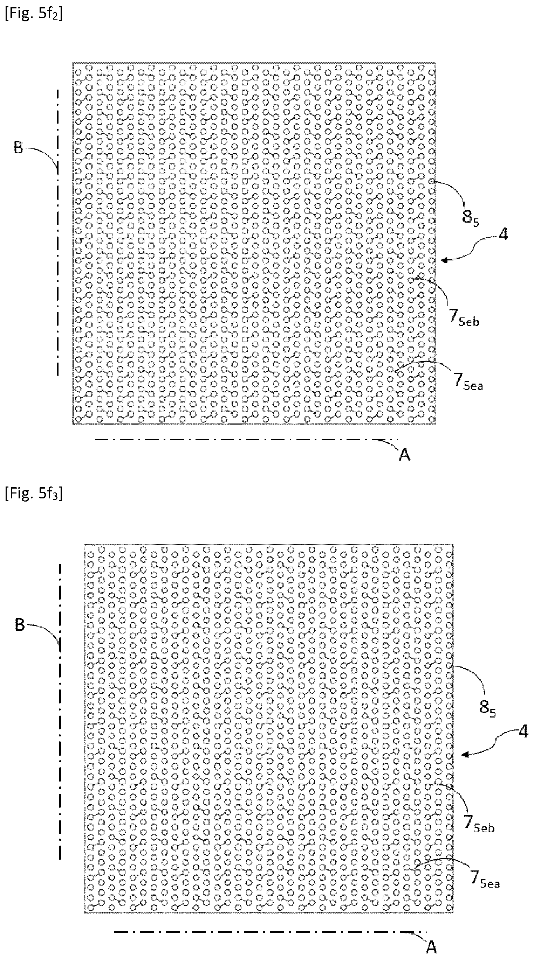

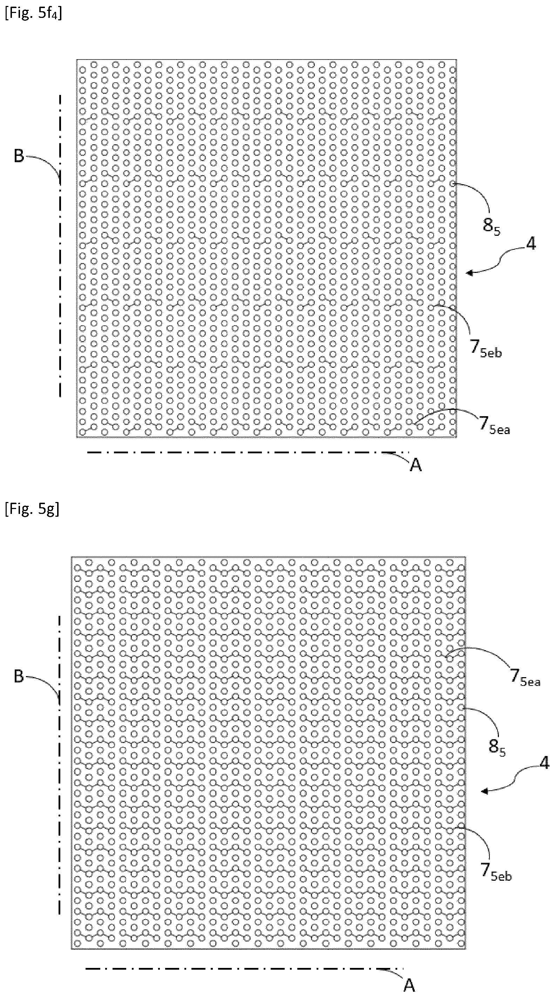

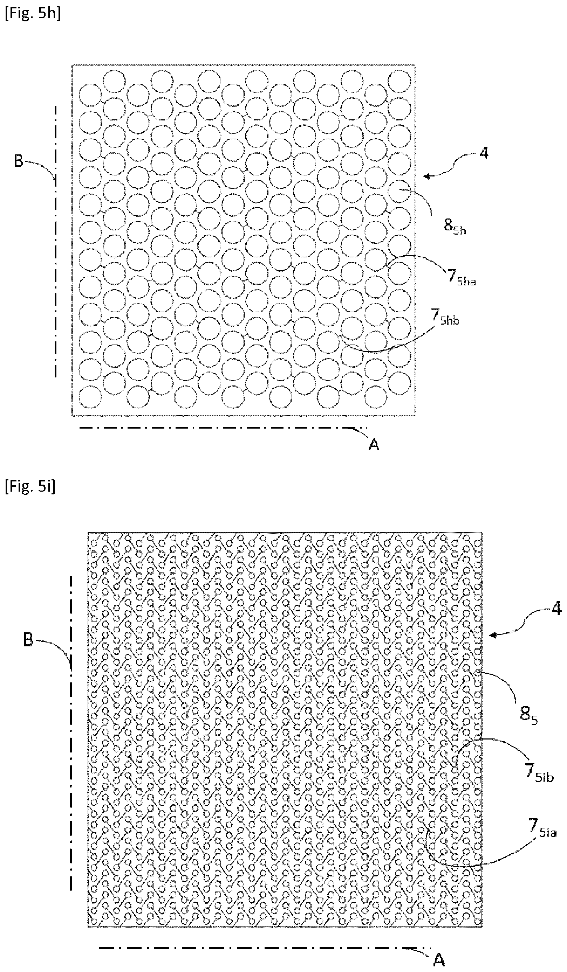

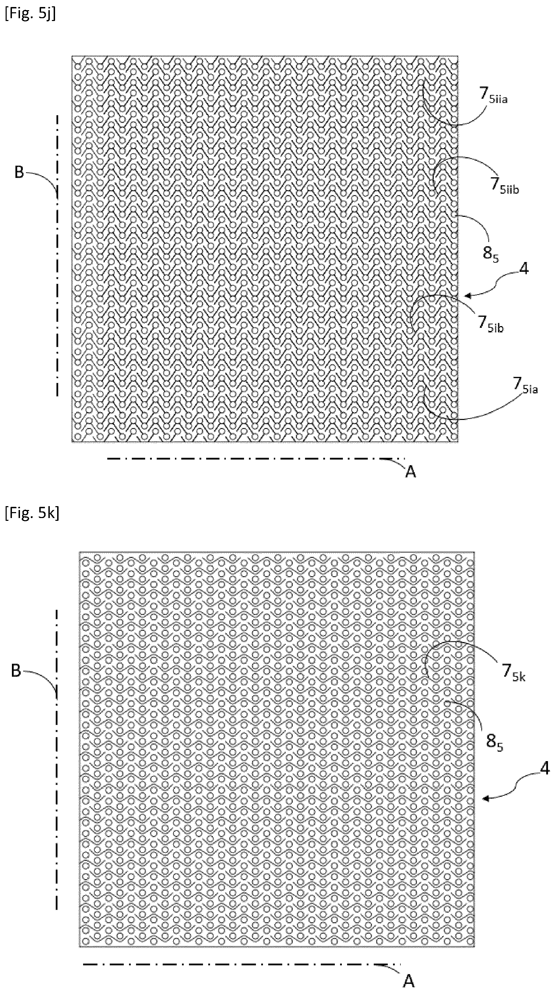

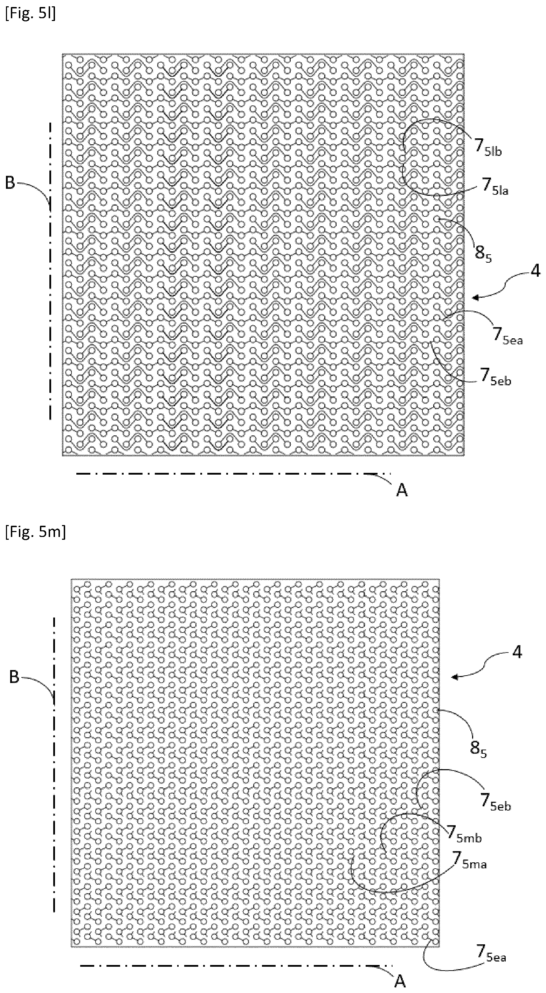

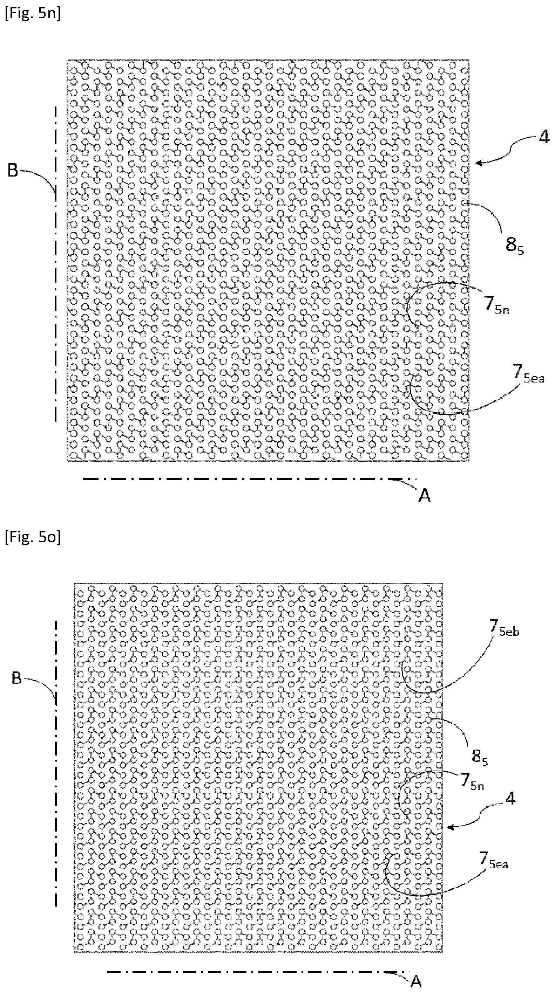

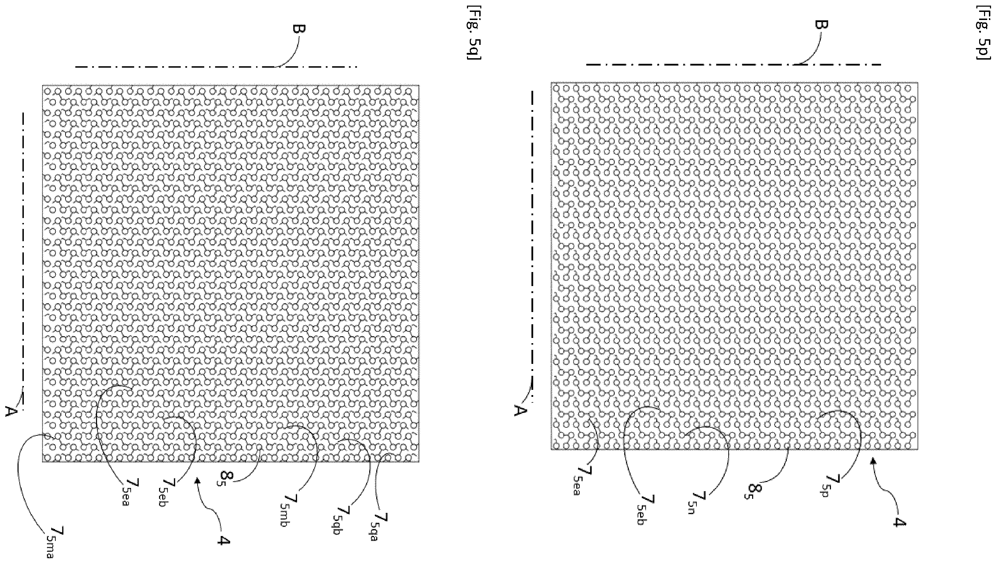

[Fig. 5b₁] La figure 5b₁ est une vue du dessus d'un 2^{ème} échantillon test de 1^{er} film mince et souple de polyuréthane.
[Fig. 5b₂] La figure 5b₂ est une vue du dessus d'un 3^{ème} échantillon test de 1^{er} film mince et souple de polyuréthane.
[Fig. 5b₃] La figure 5b₃ est une vue du dessus d'un 4^{ème} échantillon test de 1^{er} film mince et souple de polyuréthane.
[Fig. 5c] La figure 5c est une vue du dessus d'un 5^{ème} échantillon test de 1^{er} film mince et souple de polyuréthane.
[Fig. 5d] La figure 5d est une vue du dessus d'un 6^{ème} échantillon test de 1^{er} film mince et souple de polyuréthane.
[Fig. 5e₁] La figure 5e₁ est une vue du dessus d'un 7^{ème} échantillon test de 1^{er} film mince et souple de polyuréthane.
[Fig. 5e₂] La figure 5e₂ est une vue du dessus d'un 8^{ème} échantillon test de 1^{er} film mince et souple de polyuréthane.
[Fig. 5f₁] La figure 5f₁ est une vue du dessus d'un 9^{ème} échantillon test de 1^{er} film mince et souple de polyuréthane.
[Fig. 5f₂] La figure 5f₂ est une vue du dessus d'un 10^{ème} échantillon test de 1^{er} film mince et souple de polyuréthane.
[Fig. 5f₃] La figure 5f₃ est une vue du dessus d'un 11^{ème} échantillon test de 1^{er} film mince et souple de polyuréthane.
[Fig. 5f₄] La figure 5f₄ est une vue du dessus d'un 12^{ème} échantillon test de 1^{er} film mince et souple de polyuréthane.
[Fig. 5g] La figure 5g est une vue du dessus d'un 13^{ème} échantillon test de 1^{er} film mince et souple de polyuréthane.
[Fig. 5h] La figure 5h est une vue du dessus d'un 14^{ème} échantillon test de 1^{er} film mince et souple de polyuréthane.
[Fig. 5i] La figure 5i est une vue du dessus d'un 15^{ème} échantillon test de 1^{er} film mince et souple de polyuréthane.
[Fig. 5j] La figure 5j est une vue du dessus d'un 16^{ème} échantillon test de 1^{er} film mince et souple de polyuréthane.
[Fig. 5k] La figure 5k est une vue du dessus d'un 17^{ème} échantillon test de 1^{er} film mince et souple de polyuréthane.
[Fig. 5l] La figure 5l est une vue du dessus d'un 18^{ème} échantillon test de 1^{er} film mince et souple de polyuréthane.
[Fig. 5m] La figure 5m est une vue du dessus d'un 19^{ème} échantillon test de 1^{er} film mince et souple de polyuréthane.
[Fig. 5n] La figure 5n est une vue du dessus d'un 20^{ème} échantillon test de 1^{er} film mince et souple de polyuréthane.
[Fig. 5o] La figure 5o est une vue du dessus d'un 21^{ème} échantillon test de 1^{er} film mince et souple de polyuréthane.
[Fig. 5p] La figure 5p est une vue du dessus d'un 22^{ème} échantillon test de 1^{er} film mince et souple de polyuréthane.
[Fig. 5q] La figure 5q est une vue du dessus d'un 23^{ème} échantillon test de 1^{er} film mince et souple de polyuréthane.

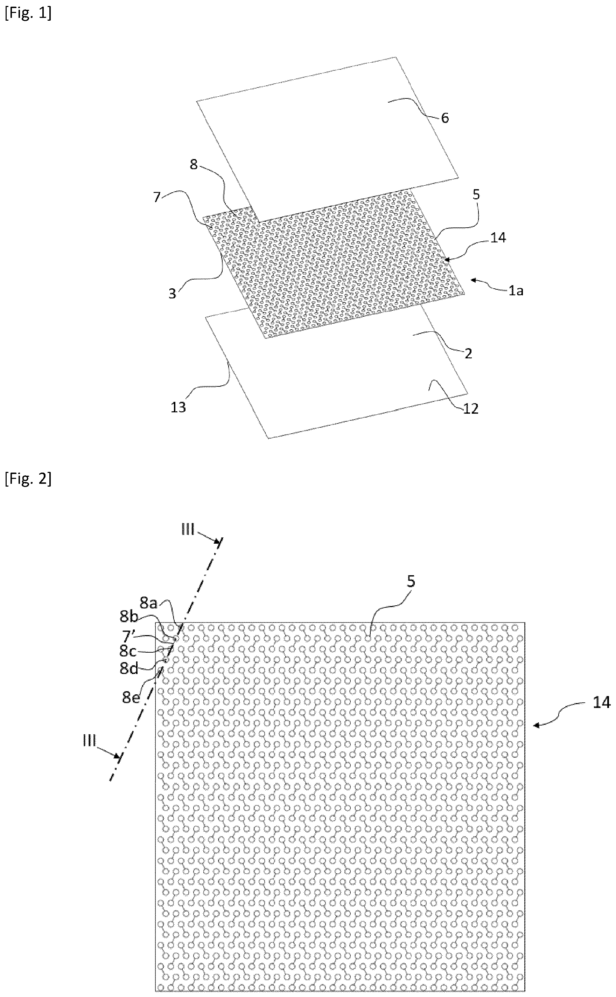

Sur la figure 1 est représentée une vue en perspective et éclatée d'un pansement à film mince 1a selon un 1^{er} mode de réalisation de l'invention.

Le pansement 1a présente une forme carrée de 150 mm de côté.

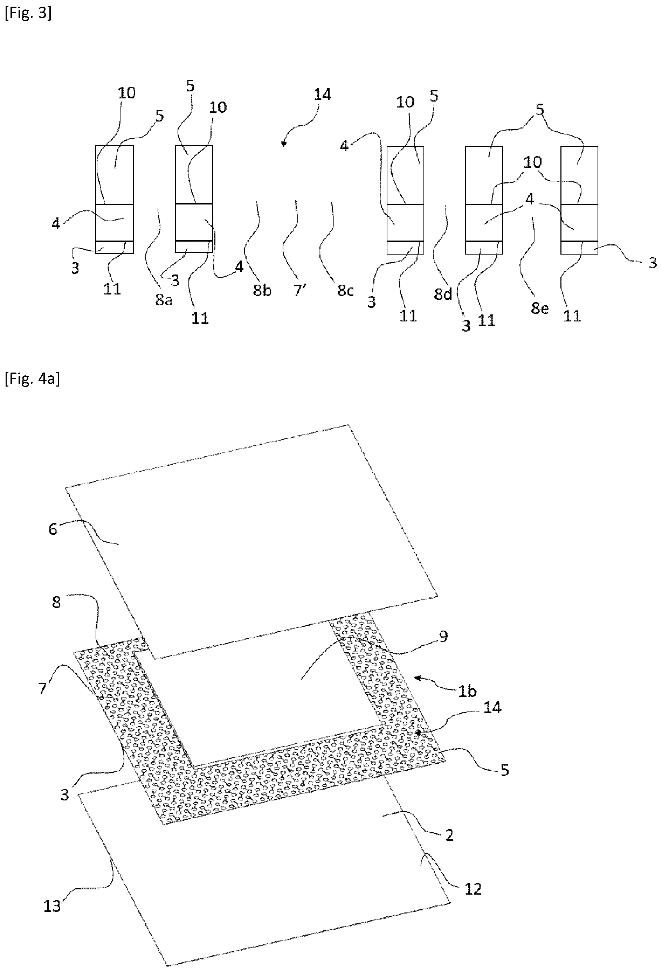

Le pansement 1a comprend un 1^{er} film mince et souple de polyuréthane 4 (visible sur la figure 3) qui présente une 1^{ère} face 10 une 2^{ème} face 11. L'épaisseur de ce 1^{er} film mince et souple de polyuréthane 4 est de 60 µm.

Le pansement 1a comprend en outre une couche d'un 1^{er} adhésif 5 qui est une couche de gel de silicone et dont une des deux faces est en contact avec la 1^{ère} face 10 du 1^{er} film mince et souple de polyuréthane 4. L'épaisseur de la couche de 1^{er} adhésif 5 est de 150 µm.

Une couche de protection détachable 6 consistant en un film de polyéthylène est positionnée sur la face de la couche du 1^{er} adhésif 5 qui est opposée à celle qui est en contact avec la 1^{ère} face 10 du 1^{er} film mince et souple de polyuréthane 4. L'épaisseur de la couche de protection détachable 6 est de 50 µm.

Le pansement 1a comprend en outre une couche d'un 2^{ème} adhésif 3 consistant en une couche d'un adhésif acrylique dont une des deux faces est en contact avec la 2^{ème} face 11 du 1^{er} film mince et souple de polyuréthane 4. L'épaisseur de la couche de 2^{ème} adhésif 3 est de 20 µm.

Le pansement 1a comprend en outre un 2^{ème} film mince et souple de polyuréthane 2 qui présente une 1^{ère} face 12 et une 2^{ème} face 13. La 1^{ère} face 12 de ce 2^{ème} film mince et souple de polyuréthane 2 est en contact avec la face de la couche du 2^{ème} adhésif 3 qui est opposée à celle en contact avec la 2^{ème} face 11 du 1^{er} film mince et souple de polyuréthane 4. L'épaisseur du 2^{ème} film mince et souple de polyuréthane 2 est de 15 µm.

L'ensemble 14 constitué par la couche de 1^{er} adhésif 5, le 1^{er} film mince et souple de polyuréthane 4 la couche de 2^{ème} adhésif 3 comporte des perforations 8 et des fentes 7 qui s'étendent dans toute l'épaisseur dudit ensemble 14.

La figure 2 est une vue du dessus dudit ensemble 14. Sur cette figure 2, les perforations 8a, 8b, 8c, 8d et 8e de forme circulaire, ainsi que la fente 7' sont référencées. Ces perforations 8a, 8b, 8c, 8d et 8e, ainsi que la fente 7' sont visibles sur la figure 3 décrite juste ci-dessous. La fente 7' s'étend longitudinalement entre les perforations 8b et 8c selon une direction parallèle à l'axe III-III.

La figure 3 est une vue en coupe selon l'axe III-III de la figure 2 de l'ensemble 14 qui est constitué par la couche du 2^{ème} adhésif 3, le 1^{er} film mince et souple de polyuréthane 4 et la couche de 1^{er} adhésif 5.

La figure 4a est une vue en perspective et éclatée d'un pansement à film mince 1b selon un 2^{ème} mode de réalisation de l'invention.

Le pansement 1b diffère du pansement 1a uniquement en ce qu'il comprend en outre une compresse 9 en mousse de polyuréthane d'une épaisseur de 2 mm. La compresse 9 est positionnée de manière centrée sur la couche de 1^{er} adhésif 5 du pansement 1b.

La figure 4b est une vue en perspective et éclatée d'un pansement à film mince 1c selon un 3^{ème} mode de réalisation de l'invention.

Le pansement 1c diffère du pansement 1a uniquement en ce qu'il comprend en outre une compresse 9 en mousse de polyuréthane d'une épaisseur de 2 mm. La compresse 9 est positionnée de manière centrée sur la couche de 2^{ème} adhésif 3 du pansement 1c.

La présente invention est maintenant illustrée à l'aide de la description des expérimentations suivantes qui ont été réalisées en mettant en œuvre différents tests selon les protocoles tels que détaillés ci-après.

Tout d'abord, l'extensibilité a été déterminée sur 23 échantillons tests de films minces et souples de polyuréthane de forme carrée (150 mm de côté) et d'épaisseur 60 µm, et qui comportaient tous des perforations, ainsi que des fentes à l'exception du 1^{er} échantillon test « 5a », lesdites perforations et fentes ayant été réalisées dans l'épaisseur desdits échantillons tests.

La disposition des perforations et des fentes pour chacun de ces 23 échantillons tests est représentée respectivement sur les figures 5a, 5b₁, 5b₂, 5b₃, 5c, 5d, 5e₁, 5e₂, 5f₁, 5f₂,5f₃, 5f₄, 5g, 5h, 5i, 5j, 5k, 5l, 5m, 5n, 5o, 5p et 5q.

L'échantillon test « 5a » représenté sur la figure 5a comportaient des perforations 8₅ de forme circulaire de 2,60 mm de diamètre disposées en quinconce de telle sorte que la distance entre le centre de deux perforations 8₅ consécutives sur une même rangée de perforations 8₅ s'étendant selon une direction parallèle à l'axe B était de 4,11 mm et la distance entre le centre de deux perforations 8₅ consécutives sur une même rangée de perforations 8₅ s'étendant selon une direction parallèle à l'axe A était de 8,60 mm. Cet échantillon test « 5a » était dépourvu de fentes et présentait un taux d'ouverture de 30%. Cet échantillon test « 5a » était donc un essai comparatif.

Les échantillons tests suivants décrits ci-dessous sont tous des échantillons tests selon l'invention.

L'échantillon test « 5b₁ » représenté sur la figure 5b₁ comportaient des perforations 8₅ de forme circulaire de 2,60 mm de diamètre disposées sous la forme de rangées de perforations 8₅ s'étendant selon une direction parallèle à l'axe A et de telle sorte que la distance entre les centres de deux perforations 8₅ consécutives d'une même rangée était de 4,11 mm et lesdites rangées de perforations 8₅ étaient espacées de 4,11 mm. Cet échantillon test « 5b₁ » comportait en outre des fentes 7_{5b} d'une longueur de 4,11 mm qui étaient disposées entre chaque rangée de perforations 8₅ s'étendant selon une direction parallèle à l'axe A comme cela est représenté sur la figure 5b₁. Cet échantillon test « 5b₁ » présentait un taux d'ouverture de 31,4%.

L'échantillon test « 5b₂ » représenté sur la figure 5b₂ était identique à l'échantillon test « 5b₁ » représenté sur la figure 5b₁ à l'exception qu'il comprenait en outre des fentes 7₅ₐ d'une longueur de 2,4 mm et qui étaient perpendiculaires aux fentes 7_{5b} et disposées comme cela est représenté sur la figure 5b₂. Cet échantillon test « 5b₂ » présentait un taux d'ouverture de 31,4%.

L'échantillon test « 5b₃ » représenté sur la figure 5b₃ était identique à l'échantillon test « 5b₁ » représenté sur la figure 5b₁ à l'exception qu'il comprenait en outre des fentes 7₅ₐ d'une longueur de 2,4 mm et qui étaient perpendiculaires aux fentes 7_{5b} et disposées comme cela est représenté sur la figure 5b₃. Cet échantillon test « 5b₃ » présentait un taux d'ouverture de 31,4%.

L'échantillon test « 5c » représenté sur la figure 5c comportaient des perforations 8₅ de forme circulaire de 2,60 mm de diamètre disposées sous la forme de rangées de perforations 8₅ s'étendant selon une direction parallèle à l'axe A et de telle sorte que la distance entre les centres de deux perforations 8₅ consécutives d'une même rangée était de 4,11 mm et lesdites rangées de perforations 8₅ étaient espacées de 4,11 mm. Cet échantillon test « 5c » comportait en outre des fentes 7_{5c} d'une longueur chacune de 1,5 mm qui reliaient ensemble deux perforations 8₅ consécutives d'une même rangée de perforations 8₅ s'étendant selon une direction parallèle à l'axe A, et ce comme cela est représenté sur la figure 5c. Cet échantillon test « 5_{c} » présentait un taux d'ouverture de 31,4%.

L'échantillon test « 5d » représenté sur la figure 5d était identique à l'échantillon test représenté « 5_{c} » sur la figure 5c à l'exception qu'il comportait des fentes 7_{5c} d'une longueur chacune de 1,5 mm qui reliaient ensemble 3 perforations 8₅ consécutives d'une même rangée de perforations 8₅ s'étendant selon une direction parallèle à l'axe A, et ce comme cela est représenté sur la figure 5d. Cet échantillon test « 5d » présentait un taux d'ouverture de 31,4%.

L'échantillon test « 5e₁ » représenté sur la figure 5e₁ était identique à l'échantillon test « 5a » représenté la figure 5a en ce qui concerne les perforations 8₅ et leur disposition. Cet échantillon test « 5e₁ » comportait en outre des 1^{ères} fentes 7₅ₑₐ d'une longueur de 2 mm et des 2^{èmes} fentes 7_{5eb} d'une longueur de 2 mm qui étaient disposées comme cela est représenté sur la figure 5e₁. Cet échantillon test « 5e₁ » présentait un taux d'ouverture de 30%.

L'échantillon test « 5e₂ » représenté sur la figure 5e₂ était identique à celui de la figure 5e₁ en ce qui concerne les perforations 8₅ et leur disposition. Cet échantillon test « 5e₂ » comportait en outre des 1^{ères} fentes 7₅ₑₐ d'une longueur de 2 mm et des 2^{èmes} fentes 7_{5eb} d'une longueur de 2 mm qui étaient disposées comme cela est représenté sur la figure 5e₂. Ainsi, certaines des fentes 7₅ₑₐ et 7_{5eb} étaient plus espacées entre elles que les fentes de l'échantillon test « 5e₁ » représenté sur la figure 5e₁_{.} Cet échantillon test « 5e₂ » présentait un taux d'ouverture de 30%.

L'échantillon test « 5f₁ » représenté sur la figure 5f₁ était identique à celui de la figure 5a en ce qui concerne les perforations 8₅ et leur disposition. Cet échantillon test « 5f₁ » comportait en outre des 1^{ères} fentes 7₅ₑₐ d'une longueur de 2 mm et des 2^{èmes} fentes 7_{5eb} d'une longueur de 2 mm qui étaient disposées comme cela est représenté sur la figure 5f₁. Les fentes 7₅ₑₐ et 7_{5eb} formaient sur 4 colonnes consécutives de perforations 8₅ un motif de « chevrons » s'étendant selon une direction parallèle à l'axe B. Cet échantillon test « 5f₁ » présentait un taux d'ouverture de 30%.

L'échantillon test « 5f₂ » représenté sur la figure 5f₂ était identique à celui de la figure 5f₁ à l'exception que les fentes 7₅ₑₐ et 7_{5eb} qui formaient sur 4 colonnes consécutives de perforations 8₅ un motif de « chevrons » s'étendant selon une direction parallèle à l'axe B étaient plus espacées entre elles que les fentes de l'échantillon test « 5f₁ » de la figure 5f₁. Cet échantillon test « 5f₂ » présentait un taux d'ouverture de 30%.

L'échantillon test « 5f₃ » représenté sur la figure 5f₃ était identique à celui de la figure 5f₂ à l'exception que les fentes 7₅ₑₐ et 7_{5eb} qui formaient sur 4 colonnes consécutives de perforations 8₅ un motif de « chevrons » s'étendant selon une direction parallèle à l'axe B étaient plus espacées entre elles que les fentes de l'échantillon test « 5f₂ » de la figure 5f₂. Cet échantillon test « 5f₃ » présentait un taux d'ouverture de 30%.

L'échantillon test « 5f₄ » représenté sur la figure 5f₄ était identique à celui de la figure 5f₃ à l'exception que les fentes 7₅ₑₐ et 7_{5eb} qui formaient sur 4 colonnes consécutives de perforations 8₅ un motif de « chevrons » s'étendant selon une direction parallèle à l'axe B étaient plus espacées entre elles que dans l'échantillon test « 5f₃ » de la figure 5f₃. Cet échantillon test « 5f₄» présentait un taux d'ouverture de 30%.

L'échantillon test « 5g » représenté sur la figure 5g était identique à celui de la figure 5a en ce qui concerne les perforations 8₅ et leur disposition. Cet échantillon test « 5g » comportait en outre des 1^{ères} fentes 7₅ₑₐ et des 2^{èmes} fentes 7_{5eb} d'une longueur chacune de 2 mm qui reliaient ensemble 4 perforations 8₅ consécutives et qui étaient disposées comme cela est représenté sur la figure 5g. Cet échantillon test « 5g » présentait un taux d'ouverture de 30%.

L'échantillon test « 5h » représenté sur la figure 5h comportaient des perforations 8₅ₕ de forme circulaire de 10 mm de diamètre disposées en quinconce de telle sorte que la distance entre le centre de deux perforations 8₅ₕ consécutives sur une même rangée de perforations 8₅ₕ s'étendant selon une direction parallèle à l'axe B était de 1,2 mm et la distance entre le centre de deux perforations 8₅ₕ consécutives sur une même rangée de perforations 8₅ₕ s'étendant selon une direction parallèle à l'axe A était de 20 mm. Cet échantillon « 5h » comportait en outre des 1^{ères} fentes 7₅ₕₐ d'une longueur de 2 mm et des 2^{èmes} fentes 7_{5hb} d'une longueur de 2 mm qui étaient disposées comme cela est représenté sur la figure 5h. Les fentes 7₅ₕₐ et 7_{5hb} formaient sur 4 colonnes consécutives de perforations 8₅ₕ un motif de « chevrons » s'étendant selon une direction parallèle à l'axe B. Cet échantillon test « 5h » présentait un taux d'ouverture de 62,9%.

L'échantillon test « 5i » représenté sur la figure 5i était identique à celui de la figure 5a en ce qui concerne les perforations 8₅ et leur disposition. Cet échantillon test « 5i » comportait en outre des 1^{ères} fentes 7₅ᵢₐ et des 2^{èmes} fentes 7_{5ib} qui étaient disposées comme cela est représenté sur la figure 5i. Cet échantillon test « 5i » présentait un taux d'ouverture de 30%.

L'échantillon test « 5j » représenté sur la figure 5j était identique à celui de la figure 5a en ce qui concerne les perforations 8₅ et leur disposition. Cet échantillon test « 5j » comportait en outre des 1^{ères} fentes 7₅ᵢₐ, des 2^{èmes} fentes 7_{5ib}, des 3^{èmes} fentes 7₅ᵢᵢₐ et des ₄^{èmes} fentes 7_{5iib} qui étaient disposées comme cela est représenté sur la figure 5j. Cet échantillon test « 5j » présentait un taux d'ouverture de 30%.

L'échantillon test « 5k » représenté sur la figure 5k était identique à celui de la figure 5a en ce qui concerne les perforations 8₅ et leur disposition. Cet échantillon test « 5k » comportait en outre des fentes 7₅ₖ qui étaient disposées comme cela est représenté sur la figure 5k. Cet échantillon test « 5k » présentait un taux d'ouverture de 30%.

L'échantillon test « 5l » représenté sur la figure 5l était identique à celui de la figure 5a en ce qui concerne les perforations 8₅ et leur disposition. Cet échantillon test « 5l » comportait en outre des 1^{ères} fentes 7₅ₑₐ, des 2^{èmes} fentes 7_{5eb}, des 3^{èmes} fentes 7₅ₗₐ et des ₄^{èmes} fentes 7_{5lb} qui étaient disposées comme cela est représenté sur la figure 5l. Cet échantillon test « 5l » présentait un taux d'ouverture de 30%.

L'échantillon test « 5m » représenté sur la figure 5m était identique à celui de la figure 5a en ce qui concerne les perforations 8₅ et leur disposition. Cet échantillon test « 5m » comportait en outre des 1^{ères} fentes 7₅ₑₐ, des 2^{èmes} fentes 7_{5eb}, des 3^{èmes} fentes 7₅ₘₐ et des ₄^{èmes} fentes 7_{5mb} qui étaient disposées comme cela est représenté sur la figure 5m. Cet échantillon test « 5m » présentait un taux d'ouverture de 30%.

L'échantillon test « 5n » représenté sur la figure 5n était identique à celui de la figure 5a en ce qui concerne les perforations 8₅ et leur disposition. Cet échantillon test « 5m » comportait en outre des 1^{ères} fentes 7₅ₑₐ et des 2^{èmes} fentes 7₅ₙ qui étaient disposées comme cela est représenté sur la figure 5n. Cet échantillon test « 5n » présentait un taux d'ouverture de 30%.

L'échantillon test « 5o » représenté sur la figure 5o était identique à celui de la figure 5a en ce qui concerne les perforations 8₅ et leur disposition. Cet échantillon test « 5o » comportait en outre des 1^{ères} fentes 7₅ₑₐ, des 2^{èmes} fentes 7_{5eb} et des 3^{èmes} fentes 7₅ₙ qui étaient disposées comme cela est représenté sur la figure 5o. Cet échantillon test « 5o » présentait un taux d'ouverture de 30%.

L'échantillon test « 5p » représenté sur la figure 5m était identique à celui de la figure 5a en ce qui concerne les perforations 8₅ et leur disposition. Cet échantillon test « 5p » comportait en outre des 1^{ères} fentes 7₅ₑₐ, des 2^{èmes} fentes 7_{5eb}, des 3^{èmes} fentes 7₅ₙ et des ₄^{èmes} fentes 7₅ₚ qui étaient disposées comme cela est représenté sur la figure 5p. Cet échantillon test « 5p » présentait un taux d'ouverture de 30%.

L'échantillon test « 5q » représenté sur la figure 5q était identique à celui de la figure 5a en ce qui concerne les perforations 8₅ et leur disposition. Cet échantillon test « 5q » comportait en outre des 1^{ères} fentes 7₅ₑₐ, des 2^{èmes} fentes 7_{5eb}, des 3^{èmes} fentes 7₅ₘₐ, des ₄^{èmes} fentes 7_{5mb}, des 5^{èmes} fentes 7_{5qa} et des 6^{èmes} fentes 7_{5qb} qui étaient disposées comme cela est représenté sur la figure 5q. Cet échantillon test « 5m » présentait un taux d'ouverture de 30%.

L'extensibilité des 23 échantillons tests a été mesurée de la manière suivante.

Chaque échantillon test a été découpé de 3 façons différentes de manière à obtenir :
- un « 1^{er} échantillon test découpé » présentant une largeur de 25 mm selon l'axe B et une longueur de 150 mm selon l'axe A ;
- un « 2^{ème} échantillon test découpé » présentant une largeur de 25 mm selon l'axe A et une longueur de 150 mm selon l'axe B ;
- un « 3^{ème} échantillon test découpé » présentant une largeur de 25 mm formant un angle de 45° avec l'axe B et une longueur de 150 mm formant un angle de 45° avec l'axe A.

La mesure de l'extensibilité a été réalisée selon la norme EN 13726-4. L'extensibilité est la force maximale, exprimée en N/cm, qui est nécessaire pour étirer de 20% :
- ledit « 1^{er} échantillon test découpé » selon une direction parallèle à l'axe A (ci-après désignée « extensibilité à 0° ») ;
- ledit « 2^{ème} échantillon test découpé » selon une direction perpendiculaire à l'axe A (ci-après désignée « extensibilité à 90° ») ;
- ledit « 3^{ème} échantillon test découpé » selon une direction formant un angle de 45° avec l'axe A (ci-après désignée « extensibilité à 45° »).

Ainsi, un échantillon test est d'autant plus conformable que l'extensibilité est la plus faible possible dans les 3 directions précitées.

Ensuite, la facilité de pose de différents pansements selon l'invention et d'un pansement comparatif a été déterminée.

Plus précisément, 23 types de pansement de forme carrée de 100 mm de côté ont été fabriqués de manière à ce que chacun des 23 types de pansement comporte un des 23 échantillons tests des films minces et souples de polyuréthane décrits ci-dessus. La 1^{ère} face de chacun de ces 23 échantillons tests des films minces et souples de polyuréthane a été recouverte d'une couche de 1^{er} adhésif consistant en du gel de silicone et d'une épaisseur de 150 µm et la 2^{ème} face a été recouverte d'une couche d'un 2^{ème} adhésif consistant en une couche d'adhésif acrylique d'une épaisseur de 20 µm. Cette couche d'acrylique était elle-même recouverte d'un 2^{ème} film mince et souple de polyuréthane d'une épaisseur de 15 µm. La couche de gel de silicone était recouverte d'une couche de protection détachable en polyéthylène téréphtalate.

L'ensemble constitué par la couche de 1^{er} adhésif, le 1^{er} film mince et souple de polyuréthane et la couche de 2^{ème} adhésif comportait des perforations et des fentes qui s'étendaient dans toute l'épaisseur dudit ensemble. Les caractéristiques et la disposition de ces perforations et fentes ont été décrites ci-dessus pour les 23 échantillons tests.

Les pansements testés au cours de cette expérimentation différaient entre eux uniquement en ce qui concerne la répartition des perforations et des fentes (le cas échéant l'absence de fentes pour le 1^{er} échantillon test) dans l'ensemble constitué par le 1^{er} film mince et souple de polyuréthane et les couches de 1^{er} et 2^{ème} adhésifs. Le pansement comparatif était celui obtenu à partir de l'échantillon test « 5a » décrit ci-dessus. Tous les autres pansements de cette expérimentation étaient des pansements selon l'invention.

La facilité de pose de ces pansements testés a été déterminée de la manière suivante : après avoir retiré la couche de protection détachable, trois exemplaires de chaque pansement testé ont été appliqués sur le poignet de 5 utilisateurs. La pose a été évaluée par des notes comprises entre 0 et 4 donnée par les utilisateurs. Une note de « 0 » correspondait à une pose très difficile du pansement testé. Une note de « 4 » correspondait à une pose très facile du pansement testé. Pour chaque pansement testé, une moyenne des notes a été calculée.

Ensuite, les pourcentages de décollement de pansements selon l'invention et d'un pansement comparatif ont été déterminés. Il s'agissait de pansements testés similaires à ceux utilisés pour les expérimentations de facilité de pose des pansements à l'exception que ces pansements comprenaient en outre une compresse de forme carrée de 50 mm de côté et d'une épaisseur de 2 mm qui était constituée de mousse de polyuréthane. Cette compresse était fixée de manière centrée sur la face de la couche de 1^{er} adhésif qui était opposée à celle qui était en contact avec la 1^{ère} face du film mince et souple de polyuréthane. Le pansement comparatif était le pansement obtenu à partir de l'échantillon test « 5a » et les autres pansements étaient des pansements selon l'invention.

Le pourcentage de décollement de ces pansements testés a été déterminé de la manière suivante : après avoir retiré la couche de protection détachable, 2 exemplaires de chaque pansement testé ont été appliqués sur les biceps droit et gauche de 5 utilisateurs. Après 12 heures de pose, on a mesuré le pourcentage de surface décollée des 2 pansements appliqués et calculé une moyenne de ces 2 pourcentages. On a également relevé si le décollement engendrait une rupture de la barrière bactérienne, à savoir si le décollement était présent de la bordure du pansement jusqu'à la compresse.

Le tableau 1 ci-dessous récapitule tous les résultats obtenus de :
- l'extensibilité à 0°, 45° et 90° pour les différents échantillons tests découpés précités ;
- la facilité de pose des 23 pansements décrits ci-dessus pour cette expérimentation ;
- le pourcentage de décollement des 23 pansements décrits ci-dessus pour cette expérimentation.

**Tableau 1**

| **Echantillon test/ Pansement comprenant échantillon test** | **Extensibilité (N/cm)** | | | **Facilité de pose** | **% de décollement** |
|---|---|---|---|---|---|
| | **0°** | **45°** | **90°** | | |
| **5a** | 1,5 | 1,4 | 1,8 | 1,2 | 29 |
| **5b₁** | 1,4 | 0,9 | 0,8 | 2,7 | 10 |
| **5b₂** | 0,9 | 0,8 | 0,8 | 2,2 | 15 |
| **5b₃** | 0,9 | 1 | 0,9 | 2,1 | 22 |
| **5c** | 1,5 | 0,3 | 0,8 | 2,9 | 11 |
| **5d** | 1,6 | 0,2 | 0,5 | 2,7 | 7 |
| **5e₁** | 0,7 | 0,5 | 0,05 | 3,8 | 5 |
| **5e₂** | 0,9 | 0,6 | 0,2 | 3,4 | 8 |
| **5f₁** | 1 | 0,2 | 0,9 | 3,3 | 10 |
| **5f₂** | 1,2 | 0,9 | 0,9 | 2,5 | 14 |
| **5f₃** | 1,3 | 1 | 0,9 | 2,1 | 20 |
| **5f₄** | 1,5 | 1,1 | 1,1 | 1,8 | 24 |
| **5g** | 1 | 0,1 | 0,5 | 3,2 | 12 |
| **5h** | 0,4 | 0,3 | 0,3 | 2,9 | 60 |
| **5i** | 0 | 0 | 0 | 3,6 | 3 |
| **5j** | 0 | 0 | 0 | 3,7 | 2 |
| **5k** | 0,8 | 0 | 0 | 3,4 | 8 |
| **5l** | 0,4 | 0 | 0 | 3,3 | 5 |
| **5m** | 0,3 | 0,5 | 0 | 3,1 | 7 |
| **5n** | 0,7 | 0,4 | 0 | 3,5 | 5 |
| **5o** | 0,4 | 0,5 | 0 | 3 | 4 |
| **5p** | 0,3 | 0 | 0 | 3,9 | 2 |
| **5q** | 0,1 | 0,4 | 0,1 | 3,8 | 3 |

En comparant les résultats de l'échantillon test comparatif « 5a » avec ceux des autres échantillons tests selon l'invention, on relève que les fentes ont un effet très positif sur :
- la conformabilité (l'extensibilité de l'échantillon test « 5a » est plus faible dans les 3 directions que celle des échantillons tests selon l'invention),
- la facilité de pose du pansement (la note de 1,2 de l'échantillon test « 5a » est la plus faible des notes),
- le pourcentage de décollement du pansement. Le pourcentage de décollement de 29% de l'échantillon test « 5a » est élevé comparé aux pourcentages de décollement des pansements selon l'invention à l'exception de celui de l'échantillon test « 5h » sur lequel nous revenons plus en détail ci-dessous.

En comparant les résultats des échantillons tests « 5c » et « 5d », on relève l'effet positif lorsque les fentes sont plus longues. L'échantillon test « 5d » est plus conformable que l'échantillon test « 5c ». En effet, ses valeurs d'extensibilité à 45° et 90° sont plus faibles que celles de l'échantillon test « 5c ». En outre, son pourcentage de décollement est beaucoup moins élevé (7% versus 11%).

En comparant les résultats des échantillons tests « 5e₁ » et « 5e₂ », on relève que l'augmentation de densité des fentes a un effet positif sur la conformabilité, la facilité de pose et le pourcentage de décollement du pansement. En effet, l'échantillon test « 5e₁ » présente des valeurs d'extensibilité plus faibles dans les 3 directions (à savoir 0°, 45° et 90°) témoignant d'une meilleure conformabilité, une meilleure note quant à la facilité de pose et un pourcentage de décollement moins élevé (à savoir 5% versus 8%) que l'échantillon test « 5e₂ ».

Ces mêmes constatations quant à l'effet de la densité des fentes sont faites en comparant les résultats des échantillons tests « 5f₁ » à « 5f₄ ». En effet, on relève également pour ces échantillons tests que l'augmentation de densité des fentes a un effet positif sur la conformabilité, la facilité de pose et le pourcentage de décollement du pansement. Parmi ces 4 échantillons tests, l'échantillon test « 5f₁ » présente les valeurs d'extensibilité les plus faibles dans les 3 directions (à savoir 0°, 45° et 90°) témoignant de la meilleure conformabilité, la note la plus élevée quant à la facilité de pose et un pourcentage de décollement le moins élevé (à savoir 10% versus 14%, 20% et 24%).

En comparant les résultats des échantillons tests « 5g » et « 5f₂ » qui diffèrent entre eux par la longueur des fentes, on relève l'effet positif de l'augmentation de la longueur des fentes sur la conformabilité, la facilité de pose et le pourcentage de décollement du pansement. En effet, l'échantillon test « 5g » présente :
- des valeurs d'extensibilité dans les 3 directions plus faibles que celles de l'échantillon test « 5f₂ » témoignant ainsi d'une meilleure conformabilité de l'échantillon test « 5g »,
- une meilleure note de facilité de pose du pansement (3,2 versus 2,5),
- un pourcentage de décollement du pansement moins élevé (12% versus 14%).

En comparant les résultats des échantillons tests « 5h » et « 5f₂ » qui diffèrent entre eux de par le dimensionnement des perforations, on relève l'effet de ce dimensionnement sur le pourcentage de décollement du pansement. En effet, l'échantillon test « 5h » comportant des perforations de bien plus grand diamètre que celui des perforations de l'échantillon test « 5f₂ » présente un pourcentage de décollement de 60% qui est bien plus élevé que celui de l'échantillon test « 5f₂ » (14%).

Parmi tous les pansements selon l'invention testés, le pansement de l'échantillon test « 5j » présente une excellente conformabilité, une très bonne facilité de pose (avec une note de 3,7) et un très faible pourcentage de décollement de 2%. Le pansement selon l'invention de l'échantillon test « 5p » présente aussi d'excellents résultats avec une très bonne conformabilité, une excellente facilité de pose (avec une note de 3,9) et un très faible pourcentage de décollement de 2%. Cela témoigne que les caractéristiques des fentes et des perforations de ces échantillons test « 5j » et « 5p », ainsi que leur disposition sont particulièrement appropriées pour rendre le pansement selon l'invention très conformable, facile à poser et sans risque de décollement.

Enfin, compte tenu de la diversité des caractéristiques des fentes et des perforations, ainsi que de la diversité de leur disposition qui ont été mises en œuvre avec ces 22 échantillons tests selon l'invention, on relève avec ces résultats que le pansement selon l'invention présente l'avantage d'avoir une conformabilité, une facilité de pose et un pourcentage de décollement qui peuvent être modulés pour répondre au mieux aux besoins de son utilisateur.

La respirabilité (autrement dit la perméabilité à la vapeur d'eau) des pansements des échantillons tests « 5a » et « 5e₁ » mis en œuvre pour l'expérimentation de détermination de facilité de pose a été déterminée en effectuant des essais selon la norme NF EN 13726 - 2, et ce en coupelle droite (le pansement testé était au contact de vapeur d'eau) et en coupelle inversée (le pansement testé était au contact de liquide).

Il s'agissait ainsi de comparer la respirabilité d'un pansement selon l'invention (à savoir le pansement comprenant l'échantillon test « 5e₁») avec celle du pansement comparatif (à savoir le pansement comprenant l'échantillon test « 5a »). Pour mémoire, l'échantillon test « 5e₁ » était identique à l'échantillon test « 5a » en ce qui concerne les perforations 8₅ et leur disposition et il comportait en outre des 1^{ères} fentes 7₅ₑₐ d'une longueur de 2 mm et des 2^{èmes} fentes 7_{5eb} d'une longueur de 2 mm.

De plus, la respirabilité avec élongation de ces pansements a été déterminée selon la norme NF EN 13726 - 2, et ce uniquement en coupelle droite (le pansement testé était au contact de vapeur d'eau) en étirant de 20% le pansement selon les directions 0° et 90° telles que définies ci-dessus pour l'expérimentation de la mesure de l'extensibilité.

La mise en œuvre de cette expérimentation de la respirabilité avec élongation est intéressante, car représentative des situations d'efforts musculaires (par exemple lors d'activités sportives) dans lesquelles le pansement est davantage déformé (en comparaison avec le repos) et le corps produit de la transpiration. C'est pourquoi, plus la respirabilité avec élongation du pansement est élevée, meilleure est son aptitude à évacuer la sueur sortant des pores de la parcelle de peau recouverte par le pansement lors de situations d'efforts de l'utilisateur.

Le tableau 2 ci-dessous récapitule les résultats de la respirabilité et de la respirabilité avec élongation pour les 2 pansements précités. Les résultats sont exprimés en g/m²/24h. Il s'agit en effet de déterminer la quantité d'eau (en g) qui s'évapore d'un m² du pansement testé en 24 heures. Plus cette quantité est élevée, meilleure est la respirabilité du pansement testé.

**Tableau 2**

| **Pansement comprenant échantillon test** | **Respirabilité** - **coupelle inversée (g/m²/24h)** | **Respirabilité** - **coupelle droite (g/m²/24h)** | **Respirabilité avec 20% d'élongation - coupelle droite (g/m²/24h)** |
|---|---|---|---|
| 5a | 9025 | 1660 | 2074 |
| **5e₁** | 12540 | 1941 | 2488 |

Au vu des résultats détaillés dans le tableau 2 ci-dessus, on relève que la respirabilité (avec ou sans élongation) du pansement selon l'invention est meilleure que celle du pansement comparatif. La présence de fentes améliore ainsi aussi la respirabilité du pansement selon l'invention.

## Revendications

1. Pansement à film mince (1a,1b,1c) comprenant au moins :
- un 1^{er} film mince et souple de polymère ou copolymère (4) présentant une 1^{ère} face (10) et une 2^{ème} face (11) ;
- une couche d'un 1^{er} adhésif (5) dont une des deux faces est en contact avec la 1^{ère} face (10) du 1^{er} film mince et souple de polymère ou copolymère (4) ;
- une couche de protection détachable (6) positionnée sur la face de la couche du 1^{er} adhésif (5) qui est opposée à celle qui est en contact avec la 1^{ère} face (10) du 1^{er} film mince et souple de polymère ou copolymère (4) ;
**caractérisé en ce que** :
ledit pansement (1a,1b,1c) comprend en outre :
- une couche d'un 2^{ème} adhésif (3) dont une des deux faces est en contact avec la 2^{ème} face (11) du 1^{er} film mince et souple de polymère ou copolymère (4),
- un 2^{ème} film mince et souple de polymère ou copolymère (2) présentant une 1^{ère} face (12) et une 2^{ème} face (13), la 1^{ère} face (12) de ce 2^{ème} film mince et souple de polymère ou copolymère (2) étant en contact avec la face de la couche du 2^{ème} adhésif (3) qui est opposée à celle en contact avec la 2^{ème} face (11) du 1^{er} film mince et souple de polymère ou copolymère (4) ;
l'ensemble (14) constitué par la couche de 1^{er} adhésif (5), le 1^{er} film mince et souple de polymère ou copolymère (4) et la couche de 2^{ème} adhésif (3) comporte des perforations (8,8a,8b,8c,8d,8e,8₅,8₅ₕ) et des fentes (7,7',7_{5b},7₅ₐ,7_{5c},7₅ₑₐ,7_{5eb},7₅ₕₐ,7_{5hb},7₅ᵢₐ,7_{5ib},7₅ᵢᵢₐ,7_{5iib},7₅ₖ,7₅ₗₐ,7_{5lb,}7₅ₘₐ,7_{5mb},7₅ₙ,7₅ₚ,7_{5qa},7_{5qb}) qui s'étendent dans toute l'épaisseur dudit ensemble (14).

2. Pansement à film mince (1a,1b,1c) selon la revendication 1, **caractérisé en ce que** l'épaisseur du 1^{er} film mince et souple de polymère ou copolymère (4) est comprise entre 15 µm et 150 µm, de préférence entre 30 µm et 100 µm et l'épaisseur du 2^{ème} film mince et souple de polymère ou copolymère (2) est comprise entre 5 µm et 50 µm, de préférence entre 10 µm et 30 µm.

3. Pansement à film mince (1a,1b,1c) selon la revendication 1 ou 2, **caractérisé en ce que** les 1^{er} et 2^{ème} films minces et souples de polymère ou copolymère (4,2) sont choisis parmi les films de polyuréthane, polyester, polyamide, polyéther, polychlorure de vinyle, polychlorure de vinylidène, d'alcools polyvinyliques, polyacétate de vinyle, polystyrène, polyoléfines (telles que polyéthylène et polypropylène), fluorure polyvinylique, les élastomères de silicone, les films de copolymère de polyéther-polyester, polyester-polyuréthane, polyéther-polyuréthane, polyéther-polyamide, les films de copolymères triblocs ou diblocs de styrène et d'oléfine (par exemple styrène/butadiène) et les films de polyéthers bloc amides.

4. Pansement à film mince (1a,1b,1c) selon l'une quelconque des revendications précédentes, **caractérisé en ce que** la couche de 1^{er} adhésif (5) est un adhésif sensible à la pression à base d'acrylique, de polyuréthane, de silicone, de caoutchouc naturel, de copolymère d'éthylène et d'acétate de vinyle ou de copolymère blocs du type poly(styrène-isoprène-styrène) ou un gel choisi parmi le gel de silicone, le gel de polyuréthane et les hydrogels.

5. Pansement à film mince (1a,1b,1c) selon l'une quelconque des revendications précédentes, **caractérisé en ce que** la couche de 2^{ème} adhésif (3) est réalisée en un matériau adhésif à base d'acrylique, de silicone, de polyuréthane, de caoutchouc naturel, d'hydrogel, de copolymère d'éthylène et d'acétate de vinyle ou de copolymère blocs du type poly(styrène-isoprène-styrène).

6. Pansement à film mince (1a,1b,1c) selon l'une quelconque des revendications précédentes, **caractérisé en ce que** le taux d'ouverture des perforations (8,8a,8b,8c,8d,8e,8₅,8₅ₕ) est compris entre 10% et 60%, de préférence entre 20% et 40%, ledit taux d'ouverture étant le pourcentage de la surface totale des perforations (8,8a,8b,8c,8d,8e,8₅,8₅ₕ) exprimé par rapport à la surface que présenterait ledit ensemble (14) en l'absence des perforations (8,8a,8b,8c,8d,8e,8₅,8₅ₕ).

7. Pansement à film mince (1a,1b,1c) selon l'une quelconque des revendications précédentes, **caractérisé en ce que** la surface de chaque perforation (8,8a,8b,8c,8d,8e,8₅,8₅ₕ) est comprise entre 0,2 mm² et 80 mm², de préférence entre 1,5 mm² et 20 mm².

8. Pansement à film mince (1a,1b,1c) selon l'une quelconque des revendications précédentes, **caractérisé en ce que** les fentes (7,7',7_{5b},7₅ₐ,7_{5c},7₅ₑₐ,7_{5eb},7₅ₕₐ,7_{5hb},7₅ᵢₐ,7_{5ib},7₅ᵢᵢₐ,7_{5iib},7₅ₖ,7₅ₗₐ,7_{5lb},7₅ₘₐ,7_{5mb},7₅ₙ,7₅ₚ,7_{5qa},7_{5qb}) ont une longueur comprise entre 0,5 mm et 20 mm, de préférence entre 1 mm et 10 mmm.

9. Pansement à film mince (1a,1b,1c) selon l'une quelconque des revendications précédentes, **caractérisé en ce que** la longueur totale des fentes (7,7',7_{5b},7₅ₐ,7_{5c},7₅ₑₐ,7_{5eb},7₅ₕₐ,7_{5hb},7₅ᵢₐ,7_{5ib},7₅ᵢᵢₐ,7_{5iib},7₅ₖ,7₅ₗₐ,7_{5lb},7₅ₘₐ,7_{5mb},7₅ₙ,7₅ₚ,7_{5qa},7_{5qb}) par unité de surface dudit ensemble (14) est comprise entre 1 mm/cm² et 30 mm/cm², de préférence entre 2 mm/cm² et 15 mm/cm².

10. Pansement à film mince (1a,1b,1c) selon l'une quelconque des revendications précédentes, **caractérisé en ce que** aucun matériau de l'ensemble (14) constitué par la couche de 1^{er} adhésif (5), le 1^{er} film mince et souple de polymère ou copolymère (4) et la couche de 2^{ème} adhésif (3) n'a été retiré lors de la formation des fentes (7,7',7_{5b},7₅ₐ,7_{5c},7₅ₑₐ,7_{5eb},7₅ₕₐ,7_{5hb},7₅ᵢₐ,7_{5ib},7₅ᵢᵢₐ,7_{5iib},7₅ₖ,7₅ₗₐ,7_{5lb},7₅ₘₐ,7_{5mb},7₅ₙ,7₅ₚ,7_{5qa},7_{5qb}) dans toute l'épaisseur dudit ensemble (14) au cours de la fabrication dudit pansement (1a,1b,1c).

11. Pansement à film mince (1a,1b,1c) selon l'une quelconque des revendications précédentes, **caractérisé en ce que** les fentes (7,7',7_{5b},7₅ₐ,7_{5c},7₅ₑₐ,7_{5eb},7₅ₕₐ,7_{5hb},7₅ᵢₐ,7_{5ib},7₅ᵢᵢₐ,7_{5iib},7₅ₖ,7₅ₗₐ,7_{5lb},7₅ₘₐ,7_{5mb},7₅ₙ,7₅ₚ,7_{5qa},7_{5qb}) relient au moins 2 perforations (8,8a,8b,8c,8d,8e,8₅,8₅ₙ) adjacentes les unes des autres, plus préférentiellement au moins 4 perforations adjacentes (8,8a,8b,8c,8d,8e,8₅,8₅ₕ) les unes des autres.

12. Pansement à film mince (1a,1b,1c) selon l'une quelconque des revendications précédentes, **caractérisé en ce que** les 1^{er} et 2^{ème} films minces et souples de polymère ou copolymère (4,2) sont en polyuréthane, la couche de 1^{er} adhésif (5) est une couche de gel de silicone et la couche de 2^{ème} adhésif (3) est une couche d'acrylique.

13. Pansement à film mince (1b,1c) selon l'une quelconque des revendications précédentes, **caractérisé en ce qu'**il comprend en outre une compresse (9) qui est fixée :
- sur la face de la couche de 1^{er} adhésif (5) qui est opposée à celle qui est en contact avec la 1^{ère} face (10) du 1^{er} film mince et souple de polymère ou copolymère (4) ou
- sur la face de la couche de 2^{ème} adhésif (3) qui est opposée à celle qui est en contact avec la 2^{ème} face (11) du 1^{er} film mince et souple de polymère ou copolymère (4).

14. Procédé de fabrication d'un pansement (1a) selon l'une quelconque des revendications 1 à 12, **caractérisé en ce qu'**il comprend au moins les étapes suivantes :
a) la 1^{ère} face (10) du 1^{er} film mince et souple de polymère ou copolymère (4) est recouverte de la couche de 1^{er} adhésif (5) et la 2^{ème} face (11) du 1^{er} film mince et souple de polymère ou copolymère (4) est recouverte de la couche de 2^{ème} adhésif (3) ;
b) une 1^{ère} couche de support est appliquée sur la face de la couche du 1^{er} adhésif (5) qui est opposée à celle qui est en contact avec la 1^{ère} face (10) du 1^{er} film mince et souple de polymère ou copolymère (4) ;
c) une 2^{ème} couche de support est appliquée sur la face de la couche du 2^{ème} adhésif (3) qui est opposée à celle qui est en contact avec la 2^{ème} face (11) du 1^{er} film mince et souple de polymère ou copolymère (4) ;
d) des perforations (8,8a,8b,8c,8d,8e,8₅,8₅ₕ) sont réalisées dans toute l'épaisseur d'un ensemble constitué par la 1^{ère} couche de support, la couche de 1^{er} adhésif (5), le 1^{er} film mince et souple de polymère ou copolymère (4), la couche de 2^{ème} adhésif (3) et la 2^{ème} couche de support ;
e) des fentes (7,7',7_{5b},7₅ₐ,7_{5c},7₅ₑₐ,7_{5eb},7₅ₕₐ,7_{5hb},7₅ᵢₐ,7_{5ib},7₅ᵢᵢₐ,7_{5iib},7₅ₖ,7₅ₗₐ,7_{5lb}, 7₅ₘₐ,7_{5mb},7₅ₙ,7₅ₚ,7_{5qa},7_{5qb}) sont réalisées dans toute l'épaisseur d'un ensemble constitué par la couche de 1^{er} adhésif (5), le 1^{er} film mince et souple de polymère ou copolymère (4), la couche de 2^{ème} adhésif (3) et la 2^{ème} couche de support ;
f) la 2^{ème} couche de support est retirée ;
g) le 2^{ème} film mince et souple de polymère ou copolymère (2) est appliqué sur la couche de 2^{ème} adhésif (3) ;
h) la 1^{ère} couche de support est retirée ;
i) la couche de protection détachable (6) est appliquée sur la couche de 1^{er} adhésif de (5) manière à obtenir ledit pansement (1a).

15. Procédé de fabrication selon la revendication 14 d'un pansement à film mince (1b,1c) selon la revendication 13, **caractérisé en ce qu'**il comprend une étape supplémentaire consistant, à l'issue de l'étape h) et avant l'étape i), à appliquer la compresse (9) :
- sur la face de la couche de 1^{er} adhésif (5) qui est opposée à celle qui est en contact avec la 1^{ère} face (10) du 1^{er} film mince et souple de polymère ou copolymère (4)
ou
- sur la face de la couche de 2^{ème} adhésif (3) qui est opposée à celle qui est en contact avec la 2^{ème} face (11) du 1^{er} film mince et souple de polymère ou copolymère (4).

## Patentansprüche

1. Dünnfolienverband (1a,1b,1c), mindestens umfassend:
- eine 1. dünne und flexible Polymer- oder Copolymerfolie (4) mit einer 1. Seite (10) und einer 2. Seite (11);
- eine Schicht eines 1. Klebstoffs (5), von der eine der beiden Seiten mit der 1. Seite (10) der 1. dünnen und flexiblen Polymer- oder Copolymerfolie (4) in Kontakt ist;
- eine ablösbare Schutzschicht (6), die auf der Seite der Schicht des 1. Klebstoffs (5) positioniert ist, die derjenigen gegenüberliegt, die mit der 1. Seite (10) der 1. dünnen und flexiblen Polymer- oder Copolymerfolie (4) in Kontakt ist;
**dadurch gekennzeichnet, dass**:
der Verband (1a, 1b, 1c) ferner Folgendes umfasst:
- eine Schicht eines 2. Klebstoffs (3), von der eine der beiden Seiten mit der 2. Seite (11) der 1. dünnen und flexiblen Polymer- oder Copolymerfolie (4) in Kontakt ist,
- eine 2. dünne und flexible Polymer- oder Copolymerfolie (2) mit einer 1. Seite (12) und einer 2. Seite (13), wobei die 1. Seite (12) der 2. dünnen und flexiblen Polymer- oder Copolymerfolie (2) mit der Seite der Schicht des 2. Klebstoffs (3) in Kontakt ist, die derjenigen gegenüberliegt, die mit der 2. Seite (11) der 1. dünnen und flexiblen Polymer- oder Copolymerfolie (4) in Kontakt ist;
die Baugruppe (14), die aus der Schicht des 1. Klebstoffs (5), der 1. dünnen und flexiblen Polymer- oder Copolymerfolie (4) und der Schicht des 2. Klebstoffs (3) besteht, Perforationen (8,8a,8b,8c,8d,8e,8₅,8₅ₕ) und Schlitze (7,7',7_{5b},7₅ₐ,7_{5c},7₅ₑₐ,7_{5eb},7₅ₕₐ,7_{5hb},7₅ᵢₐ, 7_{5ib},7₅ᵢᵢₐ,7_{5iib},7₅ₖ,7₅ₗₐ ,7_{51b},7₅ₘₐ,7_{5mb},7₅ₙ,7₅ₚ, 7_{5qa},7_{5qb}) aufweist, die sich über die gesamte Dicke der Baugruppe (14) erstrecken.

2. Dünnfolienverband (1a,1b,1c) nach Anspruch 1, **dadurch gekennzeichnet, dass** die Dicke der 1. dünnen und weichen Polymer- oder Copolymerfolie (4) zwischen 15 µm und 150 µm, vorzugsweise zwischen 30 µm und 100 µm, liegt und die Dicke der 2. dünnen und flexiblen Polymer- oder Copolymerfolie (2) zwischen 5 µm und 50 µm, vorzugsweise zwischen 10 µm und 30 µm, liegt.

3. Dünnfolienverband (1a,1b,1c) nach Anspruch 1 oder 2, **dadurch gekennzeichnet, dass** die 1. und die 2. dünne oder flexible Polymer- oder Copolymerfolie (4,2) unter Folien aus Polyurethan, Polyester, Polyamid, Polyether, Polyvinylchlorid, Polyvinylidenchlorid, Polyvinylalkoholen, Polyvinylacetat, Polystyrol, Polyolefinen (wie Polyethylen und Polypropylen), Polyvinylfluorid, Silikonelastomeren, Polyether-Polyester-, PolyesterPolyurethan-, Polyether-Polyurethan-, Polyether-Polyamid-Copolymer-Folien, Triblock- oder Diblock-Copolymer-Folien aus Styrol und Olefin (z. B. Styrol/Butadien) und Polyetherblockamid-Folien ausgewählt sind.

4. Dünnfolienverband (1a,1b,1c) nach einem der vorhergehenden Ansprüche, **dadurch gekennzeichnet, dass** die Schicht des 1. Klebstoffs (5) ein druckempfindlicher Klebstoff auf Basis von Acryl, Polyurethan, Silikon, Naturkautschuk, Ethylen- und Vinylacetat-Copolymer oder Poly(Styrol-Isopren-Styrol)-Copolymerblöcken oder ein Gel ist, das aus Silikongel, Polyurethangel und Hydrogelen ausgewählt ist.

5. Dünnfolienverband (1a,1b,1c) nach einem der vorhergehenden Ansprüche, **dadurch gekennzeichnet, dass** die Schicht des 2. Klebstoffs (3) aus einem Klebstoffmaterial auf Basis von Acryl, Silikon, Polyurethan, Naturkautschuk, Hydrogel, Ethylen- und Vinylacetat-Copolymer oder Poly(Styrol-Isopren-Styrol)-Copolymerblöcken hergestellt ist.

6. Dünnfolienverband (1a,1b,1c) nach einem der vorhergehenden Ansprüche, **dadurch gekennzeichnet, dass** die Öffnungsrate der Perforationen (8,8a,8b,8c,8d,8e,8₅,8₅ₕ) zwischen 10 % und 60 %, vorzugsweise zwischen 20 % und 40 %, liegt, wobei die Öffnungsrate der Prozentsatz der Gesamtfläche der Perforationen (8,8a,8b,8c,8d,8e,8₅ ,8₅ₕ) ist, ausgedrückt in Bezug auf die Fläche, die die Baugruppe (14) aufweisen würde, wenn keine Perforationen (8,8a,8b,8c,8d,8e,8₅,8_{5bh}) vorhanden wären.

7. Dünnfolienverband (1a,1b,1c) nach einem der vorhergehenden Ansprüche, **dadurch gekennzeichnet, dass** die Fläche jeder Perforation (8,8a,8b,8c,8d,8e,8₅,8₅ₕ) zwischen 0,2 mm² und 80 mm², vorzugsweise zwischen 1,5 mm² und 20 mm², liegt.

8. Dünnfolienverband (1a,1b,1c) nach einem der vorhergehenden Ansprüche, **dadurch gekennzeichnet, dass** die Schlitze (7,7',7_{5b},7₅ₐ,7_{5c},7₅ₑₐ,7_{5eb},7₅ₕₐ,7_{5hb},7₅ᵢₐ,7_{5ib},7₅ᵢᵢₐ,7_{5iib},7₅ₖ,7₅ₗₐ,7_{5lb},7₅ₘₐ,7_{5mb},7₅ₙ,7₅ₚ,7 _{5qa},7_{5qb}) eine Länge zwischen 0,5 mm und 20 mm, vorzugsweise zwischen 1 mm und 10 mm, aufweisen.

9. Dünnfolienverband (1a,1b,1c) nach einem der vorhergehenden Ansprüche, **dadurch gekennzeichnet, dass** die Gesamtlänge der Schlitze (7,7',7_{5b},7₅ₐ,7_{5c},7₅ₑₐ,7_{5eb},7₅ₕₐ,7_{5hb},7₅ᵢₐ,7_{5ib},7₅ᵢᵢₐ,7_{5iib},7₅ₖ,7₅ₗₐ,7_{5lb},7₅ₘₐ,7_{5mb},7₅ₙ,7₅ₚ,7 _{5qa},7_{5qb}) pro Flächeneinheit der Baugruppe (14) zwischen 1 mm/cm² und 30 mm/cm², vorzugsweise zwischen 2 mm/cm² und 15 mm/cm², liegt.

10. Dünnfolienverband (1a,1b,1c) nach einem der vorhergehenden Ansprüche, **dadurch gekennzeichnet, dass** kein Material der Baugruppe (14), bestehend aus der Schicht des 1. Klebstoffs (5), der 1. dünnen und flexiblen Polymer- oder Copolymerfolie (4) und der Schicht des 2. Klebstoffs (3), bei der Bildung der Schlitze (7,7',7_{5b},7₅ₐ,75c ,7₅ₑₐ,7_{5eb},7₅ₕₐ,7_{5hb},7₅ᵢₐ,7_{5ib},7₅ᵢᵢₐ,7_{5iib},7₅ₖ,7₅ₗₐ,7_{5lb},7₅ₘₐ,7_{5mb},7₅ₙ,7₅ₚ,7 _{5qa},7_{5qb}) über die gesamte Dicke der Baugruppe (14) während der Herstellung des Verbands (1a,1b,1c) entfernt wurde.

11. Dünnfolienverband (1a,1b,1c) nach einem der vorhergehenden Ansprüche, **dadurch gekennzeichnet, dass** die Schlitze (7,7',7_{5b},7₅ₐ,7_{5c},7₅ₑₐ,7_{5eb},7₅ₕₐ,7_{5hb},7₅ᵢₐ,7_{5ib},7₅ᵢᵢₐ,7_{5iib}, 7₅ₖ,7₅ₗₐ,7_{5lb},7₅ₘₐ,7_{5mb},7₅ₙ,7₅ₚ, 7 _{5qa},7_{5qb}) mindestens 2 aneinander angrenzende Perforationen (8,8a,8b,8c,8d,8e,8₅,8₅ₕ), bevorzugter mindestens 4 aneinander angrenzende Perforationen (8,8a,8b,8c,8d,8e,8₅,8₅ₕ), miteinander verbinden.

12. Dünnfolienverband (1a,1b,1c) nach einem der vorhergehenden Ansprüche, **dadurch gekennzeichnet, dass** die 1. und die 2. dünne und flexible Polymer- oder Copolymerfolie (4,2) aus Polyurethan bestehen, die Schicht des 1. Klebstoffs (5) eine Silikongelschicht ist und die Schicht des 2. Klebstoffs (3) eine Acrylschicht ist.

13. Dünnfolienverband (1b,1c) nach einem der vorhergehenden Ansprüche, **dadurch gekennzeichnet, dass** er ferner eine Kompresse (9) umfasst, die befestigt ist:
- auf der Seite der Schicht des 1. Klebstoffs (5), die derjenigen gegenüberliegt, die mit der 1. Seite (10) der 1. dünnen und flexiblen Polymeroder Copolymerfolie (4) in Kontakt ist, oder
- auf der Seite der Schicht des 2. Klebstoffs (3), die derjenigen gegenüberliegt, die mit der 2. Seite (11) der 1. dünnen und flexiblen Polymeroder Copolymerfolie (4) in Kontakt ist.

14. Verfahren zur Herstellung eines Verbands (1a) nach einem der Ansprüche 1 bis 12, **dadurch gekennzeichnet, dass** es mindestens die folgenden Schritte umfasst:
a) die 1. Seite (10) der 1. dünnen und flexiblen Polymer- oder Copolymerfolie (4) wird mit der Schicht des 1. Klebstoffs (5) bedeckt und die 2. Seite (11) der 1. dünnen und flexiblen Polymer- oder Copolymerfolie (4) wird mit der Schicht des 2. Klebstoffs (3) bedeckt;
b) eine 1. Trägerschicht wird auf die Seite der Schicht des 1. Klebstoffs (5) aufgetragen, die derjenigen gegenüberliegt, die mit der 1. Seite (10) der 1. dünnen und flexiblen Polymer- oder Copolymerfolie (4) in Kontakt ist;
c) eine 2. Trägerschicht wird auf die Seite der Schicht des 2. Klebstoffs (3) aufgetragen, die derjenigen gegenüberliegt, die mit der 2. Seite (11) der 1. dünnen und flexiblen Polymer- oder Copolymerfolie (4) in Kontakt ist;
d) Perforationen (8,8a,8b,8c,8d,8e,8₅,8₅ₕ) werden in der gesamten Dicke einer Baugruppe ausgebildet, die aus der 1. Trägerschicht, der Schicht des 1. Klebstoffs (5), der 1. dünnen und flexiblen Polymer- oder Copolymerfolie (4), der Schicht des 2. Klebstoffs (3) und der 2. Trägerschicht besteht;
e) Schlitze (7,7',7_{5b},7₅ₐ,7_{5c},7₅ₑₐ,7_{5eb},7₅ₕₐ,7_{5hb},7₅ᵢₐ,7_{5ib},7₅ᵢᵢₐ,7_{5iib},7₅ₖ,7₅ₗₐ,7_{5lb},7₅ₘₐ,7_{5mb},7₅ₙ,7₅ₚ, 7 _{5qa},7_{5qb}) werden in der gesamten Dicke einer Baugruppe ausgebildet, die aus der Schicht des 1. Klebstoffs (5), der 1. dünnen und flexiblen Polymer- oder Copolymerfolie (4), der Schicht des 2. Klebstoffs (3) und der 2. Trägerschicht besteht;
f) die 2. Trägerschicht wird entfernt;
g) die 2. dünne und flexible Polymer- oder Copolymerfolie (2) wird auf die Schicht des 2. Klebstoffs (3) aufgetragen;
h) die 1. Trägerschicht wird entfernt;
i) die ablösbare Schutzschicht (6) wird auf die Schicht des 1. Klebstoffs (5) aufgetragen, um den Verband (1a) zu erhalten.

15. Herstellungsverfahren nach Anspruch 14 für einen Dünnfolienverband (1b,1c) nach Anspruch 13, **dadurch gekennzeichnet, dass** es einen zusätzlichen Schritt umfasst, der darin besteht, nach Abschluss von Schritt h) und vor Schritt i) die Kompresse (9) anzubringen:
- auf der Seite der Schicht des 1. Klebstoffs (5), die derjenigen gegenüberliegt, die mit der 1. Seite (10) der 1. dünnen und flexiblen Polymeroder Copolymerfolie (4) in Kontakt ist
oder
- auf der Seite der Schicht des 2. Klebstoffs (3), die derjenigen gegenüberliegt, die mit der 2. Seite (11) der 1. dünnen und flexiblen Polymeroder Copolymerfolie (4) in Kontakt ist.

## Claims

1. A thin-film dressing (1a, 1b, 1c) comprising at least:
- a 1^{st} thin and flexible polymer or copolymer film (4) having a 1^{st} face (10) and a 2^{nd} face (11);
- a layer of a 1^{st} adhesive (5), whose one of the two faces is in contact with the 1^{st} face (10) of the 1^{st} thin and flexible polymer or copolymer film (4);
- a detachable protective layer (6) positioned on the face of the layer of the 1^{st} adhesive (5) that is opposite the one that is in contact with the 1^{st} face (10) of the 1^{st} thin and flexible polymer or copolymer film (4);
**characterized in that**:
said dressing (1a, 1b, 1c) further comprises:
- a layer of a 2^{nd} adhesive (3), whose one of the two faces is in contact with the 2^{nd} face (11) of the 1^{st} thin and flexible polymer or copolymer film (4),
- a 2^{nd} thin and flexible polymer or copolymer film (2) having a 1^{st} face (12) and a 2^{nd} face (13), the 1^{st} face (12) of this 2^{nd} thin and flexible polymer or copolymer film (2) being in contact with the face of the layer of the 2^{nd} adhesive (3) which is opposite to that in contact with the 2^{nd} face (11) of the 1^{st} thin and flexible polymer or copolymer film (4);
the assembly (14) consisting of the layer of 1^{st} adhesive (5), the 1^{st} thin and flexible polymer or copolymer film (4) and the layer of 2^{nd} adhesive (3) includes perforations (8, 8a, 8b, 8c, 8d, 8e, 8₅, 8₅ₕ) and slits (7,7', 7_{5b}, 7₅ₐ,7_{5c},7₅ₑₐ,7_{5eb},7₅ₕₐ, 7_{5hb}, 7₅ᵢₐ,7_{5ib},7₅ᵢᵢₐ,7_{5iib}, 7₅ₖ,7₅ₗₐ,7_{5lb},7₅ₘₐ,7_{5mb},7₅ₙ,7₅ₚ, 7_{5qa}, 7_{5qb}) which extend throughout the entire thickness of said assembly (14).

2. The thin-film dressing (1a, 1b, 1c) according to claim 1, **characterized in that** the thickness of the 1^{st} thin and flexible polymer or copolymer film (4) is comprised between 15 µm and 150 µm, preferably between 30 µm and 100 µm and the thickness of the 2^{nd} thin and flexible polymer or copolymer film (2) is comprised between 5 µm and 50 µm, preferably between 10 µm and 30 µm.

3. The thin-film dressing (1a, 1b, 1c) according to claim 1 or 2, **characterized in that** the 1^{st} and 2^{nd} thin and flexible polymer or copolymer films (4, 2) are selected from polyurethane, polyester, polyamide, polyether, polyvinyl chloride, polyvinylidene chloride, polyvinyl alcohol, polyvinyl acetate, polystyrene, polyolefins (such as polyethylene and polypropylene), polyvinyl fluoride, silicone elastomers, polyether-polyester, polyester-polyurethane, polyether-polyurethane, polyetherpolyamide copolymer films, styrene and olefin triblock or diblock copolymer films (for example styrene/butadiene), and polyether block amide films.

4. The thin-film dressing (1a, 1b, 1c) according to any one of the preceding claims, **characterized in that** the 1^{st} adhesive layer (5) is a pressuresensitive adhesive based on acrylic, polyurethane, silicone, natural rubber, ethylene vinyl acetate copolymer or block copolymer of the poly(styrene-isoprene-styrene) type or a gel selected from silicone gel, polyurethane gel and hydrogels.

5. The thin-film dressing (1a, 1b, 1c) according to any one of the preceding claims, **characterized in that** the 2^{nd} adhesive layer (3) is made of an adhesive material based on acrylic, silicone, polyurethane, natural rubber, hydrogel, ethylene vinyl acetate copolymer or block copolymer of the poly(styrene-isoprene-styrene) type.

6. The thin-film dressing (1a, 1b, 1c) according to any one of the preceding claims, **characterized in that** the opening rate of the perforations (8, 8a, 8b, 8c, 8d, 8e, 8₅, 8₅ₕ) is comprised between 10% and 60%, preferably between 20% and 40%, said opening rate being the percentage of the total surface area of the perforations (8, 8a, 8b, 8c, 8d, 8e, 8₅, 8₅ₕ) expressed relative to the surface area that said assembly (14) would have in the absence of the perforations (8, 8a, 8b, 8c, 8d, 8e, 8₅, 8₅ₕ).

7. The thin-film dressing (1a, 1b, 1c) according to any one of the preceding claims, **characterized in that** the surface area of each perforation (8, 8a, 8b, 8c, 8d, 8e, 8₅, 8₅ₕ) is comprised between 0.2 mm² and 80 mm², preferably between 1.5 mm² and 20 mm².

8. The thin-film dressing (1a, 1b, 1c) according to any one of the preceding claims, **characterized in that** the slits (7,7', 7_{5b}, 7₅ₐ,7_{5c},7₅ₑₐ,7_{5eb},7₅ₕₐ,7_{5hb}, 7₅ᵢₐ,7_{5ib}, 7₅ᵢᵢₐ,7_{5iib}, 7₅ₖ,7₅ₗₐ,7_{5lb},7₅ₘₐ,7_{5mb},7₅ₙ,7₅ₚ, 7_{5qa}, 7_{5qb}) have a length comprised between 0.5 mm and 20 mm, preferably between 1 mm and 10 mm.

9. The thin-film dressing (1a, 1b, 1c) according to any one of the preceding claims, **characterized in that** the total length of the slits (7,7', 7_{5b}, 7₅ₐ,7_{5c}, 7₅ₑₐ, 7_{5eb}, 7₅ₕₐ, 7_{5hb}, 7₅ᵢₐ, 7_{5ib}, 7₅ᵢᵢₐ, 7_{5iib}, 7₅ₖ, 7₅ₗₐ,7_{5lb},7₅ₘₐ, 7_{5mb}, 7₅ₙ, 7₅ₚ, 7_{5qa}, 7_{5qb}) per unit area of said assembly (14) is comprised between 1 mm/cm² and 30 mm/cm², preferably between 2 mm/ cm² and 15 mm/cm².

10. The thin-film dressing (1a, 1b, 1c) according to any one of the preceding claims, **characterized in that** no material from the assembly (14) consisting of the 1^{st} adhesive layer (5), the 1^{st} thin and flexible polymer or copolymer film (4) and the 2^{nd} adhesive layer (3) was removed during the formation of the slits (7,7', 7_{5b}, 7₅ₐ, 7_{5c}, 7₅ₑₐ, 7_{5eb}, 7₅ₕₐ, 7_{5hb}, 7₅ᵢₐ, 7_{5ib}, 7₅ᵢᵢₐ, 7_{5iib}, 7₅ₖ, 7₅ₗₐ,7_{5lb},7₅ₘₐ, 7_{5mb}, 7₅ₙ, 7₅ₚ, 7_{5qa}, 7_{5qb}) throughout the entire thickness of said assembly (14) during the manufacture of said dressing (1a, 1b, 1c).

11. The thin-film dressing (1a, 1b, 1c) according to any one of the preceding claims, **characterized in that** the slits (7,7', 7_{5b}, 7₅ₐ, 7_{5c}, 7₅ₑₐ, 7_{5eb}, 7₅ₕₐ, 7_{5hb}, 7₅ᵢₐ, 7_{5ib}, 7₅ᵢᵢₐ, 7_{5iib}, 7₅ₖ, 7₅ₗₐ, 7_{5lb}, 7₅ₘₐ, 7_{5mb}, 7₅ₙ, 7₅ₚ, 7_{5qa}, 7_{5qb}) connect at least 2 perforations (8, 8a, 8b, 8c, 8d, 8e, 8₅, 8₅ₕ) adjacent to each other, more preferably at least 4 adjacent perforations (8, 8a, 8b, 8c, 8d, 8e, 8₅, 8₅ₕ) to each other.

12. The thin-film dressing (1a, 1b, 1c) according to any one of the preceding claims, **characterized in that** the 1^{st} and 2^{nd} thin and flexible polymer or copolymer films (4, 2) are made of polyurethane, the layer of the 1^{st} adhesive (5) is a layer of silicone gel and the layer of the 2^{nd} adhesive (3) is a layer of acrylic.

13. The thin film dressing (1b, 1c) according to any one of the preceding claims, **characterized in that** it further comprises a compress (9) which is fixed:
- on the face of the layer of the 1^{st} adhesive (5) which is opposite to that which is in contact with the 1^{st} face (10) of the 1^{st} thin and flexible polymer or copolymer film (4)
or
- on the face of the layer of 2^{nd} adhesive (3) which is opposite to that which is in contact with the 2^{nd} face (11) of the 1^{st} thin and flexible polymer or copolymer film (4).

14. A method for manufacturing a dressing (1a) according to any one of claims 1 to 12, **characterized in that** it comprises at least the following steps:
a) the 1^{st} face (10) of the 1^{st} thin and flexible polymer or copolymer film (4) is covered with the layer of 1^{st} adhesive (5) and the 2^{nd} face (11) of the thin and flexible polymer or copolymer film (4) is covered with the layer of 2^{nd} adhesive (3);
b) a 1^{st} support layer is applied to the face of the layer of 1^{st} adhesive (5) which is opposite to that which is in contact with the 1^{st} face (10) of the thin and flexible polymer or copolymer film (4);
c) a 2^{nd} support layer is applied to the face of the layer of 2^{nd} adhesive (3) which is opposite to that which is in contact with the 2^{nd} face (11) of the 1^{st} thin and flexible polymer or copolymer film (4);
d) perforations (8, 8a, 8b, 8c, 8d, 8e, 8₅, 8₅ₕ) are made throughout the entire thickness of an assembly consisting of the 1^{st} support layer, the 1^{st} adhesive layer (5), the 1^{st} thin and flexible polymer or copolymer film (4), the 2^{nd} adhesive layer (3) and the 2^{nd} support layer;
e) slits (7,7', 7_{5b}, 7₅ₐ,7_{5c},7₅ₑₐ,7_{5eb},7₅ₕₐ,7_{5hb}, 7₅ᵢₐ,7_{5ib}, 7₅ᵢᵢₐ,7_{5iib}, 7₅ₖ, 7₅ₗₐ, 7_{5lb},7₅ₘₐ,7_{5mb},7₅ₙ,7₅ₚ, 7_{5qa}, 7_{5qb}) are made throughout the entire thickness of an assembly consisting of the 1^{st} adhesive layer (5), the 1^{st} thin and flexible polymer or copolymer film (4), the 2^{nd} adhesive layer (3) and the 2^{nd} support layer;
f) the 2^{nd} support layer is removed;
g) the 2^{nd} thin and flexible polymer or copolymer film (2) is applied to the 2^{nd} adhesive layer (3);
h) the 1^{st} support layer is removed;
i) the removable protective layer (6) is applied to the 1^{st} adhesive layer (5) so as to obtain said dressing (1a).

15. The method for manufacturing according to claim 14 a thin-film dressing (1b, 1c) according to claim 13, **characterized in that** it comprises an additional step consisting, at the end of step h) and before step i), of applying the compress (9):
- on the face of the layer of 1^{st} adhesive (5) which is opposite to that which is in contact with the 1^{st} face (10) of the 1^{st} thin and flexible polymer or copolymer film (4)
or
- on the face of the layer of 2^{nd} adhesive (3) which is opposite to that which is in contact with the 2^{nd} face (11) of the 1^{st} thin and flexible polymer or copolymer film (4).
